# EUROPEAN PATENT APPLICATION

(11) **EP 2 347 769 A1**
(43) Date of publication of application: **27.07.2011**
(21) Application number: 10000527.1
(22) Date of filing: 20.01.2010
(51) Int. Cl.: A61K 47/48, C07K 16/30

(54) **Cancer stem cell markers and uses thereof**

(71) Applicant: Glycotope GmbH, 13125 Berlin (DE)
(72) Inventor: Goletz, Steffen, 13125 Berlin (DE); Karsten, Uwe, 16341 Panketal (DE)
(74) Representative: Roth, Carla

(57) **Abstract**

The present invention *inter alia* pertains to therapeutic methods which are based on the use of an agent specifically binding a tumor-associated carbohydrate antigen for the treatment of cancer stem cells and related diseases. Also provided are diagnostic and prognostic methods using a tumor-associated carbohydrate antigen as marker for cancer stem cells.

## Description

The present invention *inter alia* pertains to therapeutic methods which are based on the use of an agent specifically binding a tumor-associated carbohydrate antigen for the treatment of cancer stem cells and related diseases. Also provided are diagnostic and prognostic methods using a tumor-associated carbohydrate antigen as marker for cancer stem cells.

### BACKGROUND OF THE INVENTION

A tumor can be viewed as an aberrant organ initiated by a tumorigenic cancer cell that acquired the capacity for indefinite proliferation through accumulated mutations. In this view of a tumor as an abnormal organ, the principles of normal stem cell biology can be applied to better understand how tumors develop and disseminate. Many observations suggest that analogies between normal stem cells and tumorigenic cells are appropriate. Both normal stem cells and tumorigenic cells have extensive proliferative potential and the ability to give rise to new (normal or abnormal) tissues. Tumorigenic cells can be thought of as cancer stem cells (CSC) or cancer initiating cells (CIC - the terms CSC and CIC are used as synonyms herein) that undergo an aberrant and poorly regulated process of organogenesis analogous to what normal stem cells do. Both tumors and normal tissues are composed of heterogeneous combinations of cells, with different phenotypic characteristics and different proliferative potentials.

Cancer stem cells are believed to be a certain fraction of tumor cells with stem cell-like properties, which initiate and maintain neoplastic clones. These cells have the ability to self-renew, but also give rise to progenitors that yield phenotypically diverse cancer cells but with lower tumorigenic potential. This subpopulation of stem cell-like cells are the ones that are efficient at tumor formation and metastatic tumor spread as compared to tumor cells that are not cancer stem cells.

Cancer stem cells (CSCs) have now been identified in a wide variety of cancers including leukemias, glioblastomas, medulloblastomas, and almost all types of epithelial tumors (carcinomas).

The theory that cancer stem cells (CSC) usually are a prerequisite for cancer ontogenesis is now widely accepted. Cancer stem cells exhibit low proliferative rates, high self-renewing capacity, a propensity to differentiate into actively proliferating tumor cells, and show resistance to chemotherapy or radiation (see e.g. Van der Griend et al. 2008).

Cancer stem cells can be characterized based on the investigation of distinct surface marker patterns within primary tumors. Among an ever increasing number of proposed CSC markers, two are most prominent and accepted, and are also widely distributed among CSCs of different tumor types: CD44 and CD133. CD44 was reported as a robust marker of cancer stem cells (Chu et al. 2009; Takaishi et al. 2009). A single CD44* cell from a colorectal tumor could form a sphere *in vitro* and was able to generate a xenograft tumor resembling the properties of the primary tumor (Du et al. 2008). CD133 is also a marker of cancer stem cells. CD133 was initially described as a surface antigen specific for human hematopoietic stem cells and as a marker for murine neuroepithelia and several other embryonic epithelia (Singh et al. 2004). In a number of recent studies, CD133 alone or in combination with other markers was used for the isolation of cancer stem cells from malignant tumors of colon, lung and liver (Haraguchi et al. 2008). CD133⁺ tumor cells repair radiation-induced DNA damage more effectively than CD133- tumor cells (Bao et al. 2006).

The presence of cancer stem cells has profound implications for cancer therapy. Existing therapies have been developed largely against the bulk population of tumor cells, because the therapies are identified by their ability to shrink the tumor mass. However, cancer stem cells are often resistant to chemotherapy and can account for chemotherapy failure (Sell et al. 2008). To design novel therapeutic agents that (also) target cancer-initiating cells (also referred herein as cancer stem cells), it will be desirable to seek molecular targets of cancer stem cells that are preferably absent on benign tumors and/or normal non-tumor cells, and at the same time are specifically directed against cancer stem cells. Such agents are anticipated to result in more durable responses and cures of tumors, and especially of metastatic tumors. Therefore, new cancer stem cell markers are wanted to provide novel therapeutic targets to improve therapy. Most of the known stem-cell markers are proteins. Many of them have also been found to be normal stem cell markers and are thus expressed on non-tumor stem cells. This makes them not or at least less suitable as therapeutic target. At present, there is no clear cut distinction between normal and cancer stem cell markers.

Thus, it is highly desirable to be able to identify further suitable cancer stem cells markers, and to use these markers for diagnostic and prognostic methods and/or for developing therapies that target cancer stem cells.

### SUMMARY OF THE INVENTION

The present invention is based on the finding that tumor-associated carbohydrate antigens are suitable cancer stem cell markers. Since almost all cancer stem cell markers described so far are proteins, it was very surprising to find that tumor-associated carbohydrate antigens such as e.g. CD176. CD174 and CD173. are expressed on cancer stem cells. Thus, these tumor-associated carbohydrate antigens are suitable markers for cancer stem cells and furthermore, provide suitable therapeutic targets for a therapy that attacks cancer stem cells.

According to a first aspect of the present invention, a binding agent is provided which specifically binds a tumor-associated carbohydrate antigen for use in the treatment of cancer stem cells expressing said tumor-associated carbohydrate antigen. Also provided is a pharmaceutical composition, comprising a respective binding agent for use in the treatment of cancer stem cells expressing said tumor-associated carbohydrate antigen.

According to a second aspect, a method is provided for identifying a cancer comprising cancer stem cells that is susceptible to treatment with a binding agent that specifically binds a tumor-associated carbohydrate antigen wherein said treatment effects the cancer stem cells, comprising determining whether a cancer sample obtained from a patient comprises cancer stem cells that express the tumor-associated carbohydrate antigen the binding agent is specific for, wherein the presence of said tumor-associated carbohydrate antigen on cancer stem cells indicates that the cancer is susceptible to treatment with the binding agent that specifically binds said tumor-associated carbohydrate antigen and wherein said treatment effects the cancer stem cells.

According to a third aspect, a method for diagnosing, staging and/or prognosing cancer and/or monitoring the susceptibility to treatment is provided, comprising the step of analyzing the expression of a tumor-associated carbohydrate antigen on cells in a sample isolated from a patient, wherein the presence of cells expressing the tumor-associated carbohydrate antigen indicates the presence of cancer stem cells in said sample.

According to a fourth aspect, a composition of mammalian cancer stem cells is provided, wherein at least 50%, preferably at least 75% of the cells in the composition are cancer stem cells which express a tumor-associated carbohydrate antigen. According to a related aspect, a method for the isolation of cancer stem cells is provided, comprising the selective enrichment of cancer stem cells which express a tumor-associated carbohydrate antigen.

According to a fifth aspect, a kit for use in a method according to the present invention is provided, comprising a binding agent which specifically binds a tumor-associated carbohydrate antigen and instructions for use in a method according to the present invention.

According to a sixth aspect, a method is provided for screening a candidate therapeutic agent, e.g. a chemotherapeutic agent or another anti-cancer drug, for effectiveness against a cancer stem cell expressing a tumor-associated carbohydrate antigen, the method comprising:
a. contacting said agent with the cell composition according to the present invention, and
b. determining the effectiveness of said agent against said tumor-associated carbohydrate antigen positive cancer cells.

Other objects, features, advantages and aspects of the present invention will become apparent to those skilled in the art from the following description and appended claims. It should be understood, however, that the following description, appended claims, and specific examples, which indicate preferred embodiments of the application are given by way of illustration only. Various changes and modifications within the spirit and scope of the disclosed invention will become readily apparent to those skilled in the art from reading the following.

### DEFINITIONS

As used herein, the following expressions are generally intended to preferably have the meanings as set forth below, except to the extent that the context in which they are used indicates otherwise.

A "binding agent" may be any compound or complex of compounds which is capable of binding a target substance such as a tumor-associated carbohydrate antigen. Preferably, the binding agent is capable of specifically binding the target substance. Suitable binding agents may be obtained by screening a binding agent library in order to identify/obtain binding agents that bind to the target substance. Examples for respective binding agent libraries are for example phage or phagemid libraries, which display the binding agents. Methods for obtaining e.g. antibodies *in vitro* are also described by Hudson and Souriau (Hudson, P.J. and Souriau, C. (2003) "Engineered antibodies" Nat. Med. 9. 129-134).

The binding agents may have any structure, as long as they are able to specifically recognize and bind the target substance, here a tumor-associated carbohydrate antigen. Binding agents may be selected from the group consisting of antibodies, antigen-binding fragments or derivatives thereof or binding agents having a protein scaffold providing a binding function such as for example anticalins or lectins. Binding agents may also be peptides or fusion proteins providing a binding function. An overview of binding agents which have a similar binding function as antibodies is given in Hey, et al. (Hey et al. (2005) "Artificial, non-antibody binding proteins for pharmaceutical and industrial application", Trends in Biotechnology 23(10), 514-522). An antibody derivative also includes antibodies or antibody fragments having the same binding function but e.g. an altered amino acid sequence.

The term "antibody" particularly refers to a protein comprising at least two heavy chains and two light chains connected by disulfide bonds. The term "antibody" includes naturally occurring antibodies as well as all recombinant forms of antibodies, e.g., antibodies expressed in prokaryotes, non-glycosylated antibodies, humanized antibodies, and chimeric antibodies. Each heavy chain is comprised of a heavy chain variable region (V_{H}) and a heavy chain constant region (C_{H}). Each light chain is comprised of a light chain variable region (V_{L} and a light chain constant region (C_{L}). The heavy chain-constant region comprises three or - in the case of antibodies of the IgM- or IgE-type - four heavy chain-constant domains (C_{H}1, C_{H}2, C_{H}3 and C_{H}4) wherein the first constant domain C_{H}1 is adjacent to the variable region and may be connected to the second constant domain C_{H}2 by a hinge region. The light chain-constant region consists only of one constant domain. The variable regions can be further subdivided into regions of hypervariability, termed complementarity determining regions (CDRs), interspersed with regions that are more conserved, termed framework regions (FR), wherein each variable region comprises three CDRs and four FRs. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. IgA and IgM molecules normally occur with a J chain but also molecules derived therefrom e.g. without the J-chain are explicitly included.

An "antigen-binding fragment or derivative" of an antibody in particular is a protein or glycoprotein which is capable of binding an antigen, e.g. to the same epitope as a reference antibody. Thus, an antigen-binding fragment or derivative of an antibody herein generally refers to a functional, antigen-binding fragment Preferably, the antigen-binding fragment comprises at least 20 amino acids from a whole antibody, more preferably at least 100 amino acids, preferably including the binding region of an antibody. In particularly preferred embodiments, the antigen-binding fragment of an antibody comprises a heavy chain variable region. It has been shown that the antigen-binding function of an antibody can be performed by fragments of a full-length antibody. Examples of fragments of an antibody include (I) Fab fragments, monovalent fragments consisting of the variable region and the first constant domain of each the heavy and the light chain; (ii) F(ab)₂ fragments, bivalent fragments comprising two Fab fragments linked together, for example by a disulfide bridge at the hinge region; (iii) Fd fragments consisting of the variable region and the first constant domain C_{H}1 of the heavy chain; (iv) Fv fragments consisting of the heavy chain and light chain variable region of a single arm of an antibody; (v) scFv fragments, Fv fragments consisting of a single polypeptide chain; (vi) (F_{V})₂ fragments consisting of two Fv fragments covalently linked together; (vii) a heavy chain variable domain; and (viii) multibodies consisting of a heavy chain variable region and a light chain variable region covalently linked together in such a manner that association of the heavy chain and light chain variable regions can only occur intermolecular but not intramolecular. Also single domain antibodies are comprised by the term "an antibody or antigen-binding fragment or derivative thereof". Also antibodies or antibody fragments with different glycan side chains are also understood by this definition. These antibody fragments or derivatives can be obtained using conventional techniques known to those with skill in the art.

"Specific binding" preferably means that a binding agent such as an antibody or an antigen-binding fragment or derivative binds stronger to a target such as an epitope for which it is specific compared to the binding to another target. An agent binds stronger to a first target compared to a second target if it binds to the first target with a dissociation constant (K_{d}) which is lower than the dissociation constant for the second target. Preferably the dissociation constant for the target to which the agent binds specifically is more than 2-fold, preferably more than 5-fold, more preferably more than 10-fold, even more preferably more than 20-fold, 50-fold, 100-fold, 200-fold, 500-fold or 1000-fold lower than the dissociation constant for the target to which the agent does not bind specifically. If a binding agent which specifically binds to a target compound (such as CD176) is used in a particular method for binding to said target compound, the conditions for binding preferably are chosen so that binding of said binding agent to other, in particular similar compounds (such as other carbohydrate antigens) is not detectable in the method or does not interfere with the detection and/or determination of the binding to the target.

As used herein, the term "protein" in particular refers to a molecular chain of amino acids or a complex of more than one amino acid chain. A protein can contain any of the naturally occurring amino acids as well as artificial amino acids and can be of biologic or synthetic origin. A protein may be modified, naturally (post-translational modifications) or synthetically, by e.g. glycosylation, amidation, carboxylation and/or phosphorylation. A protein comprises at least two amino acids, but does not have to be of any specific length; this term does not include any size restrictions. In the present application, the terms "protein", "polypeptide" and "peptide" are used interchangeably-Preferably, a protein comprises at least 10 amino acids, preferably at least 50 amino acids, at least 100 amino acids and most preferred at least 100 amino acids.

The term "patient" means according to the invention a human being, a nonhuman primate or another animal, in particular a mammal such as a cow, horse, pig, sheep, goat, dog, cat or a rodent such as a mouse or rat. In a particularly preferred embodiment, the patient is a human being.

The term "cancer" according to the invention in particular refers to but is not limited to leukemias, seminomas, melanomas, teratomas, lymphomas, myelomas, neuroblastomas, gliomas, rectal cancer, endometrial cancer, kidney cancer, adrenal cancer, thyroid cancer, skin cancer, cancer of the brain, cervical cancer, intestinal cancer, liver cancer, colon cancer, stomach cancer, intestine cancer, head and neck cancer, gastrointestinal cancer, lymph node cancer, esophagus cancer, colorectal cancer, pancreas cancer, ear, nose and throat (ENT) cancer, breast cancer, prostate cancer, cancer of the uterus, ovarian cancer and lung cancer, and the metastases thereof. Cancer, as used herein, refers to hyperproliferative conditions. The term denotes malignant as well as non-malignant cell populations but preferably refers to malignant cells, respectively cell populations. Such disorders have an excess cell proliferation of one or more subsets of cells, which often appear to differ from the surrounding tissue both morphologically and genotypically. The excess cell proliferation can be determined by reference to the general population and/or by reference to a particular patient, e.g. at an earlier point in the patient's life. Hyperproliferative cell disorders can occur in different types of animals and in humans, and produce different physical manifestations depending upon the affected cells.

By "tumor" is meant a group of cells or tissue that is formed by misregulated cellular proliferation. Tumors may show partial or complete lack of structural organization and functional coordination with the normal tissue, and usually form a distinct mass of tissue, which may be either benign or malignant. In particular, the term tumor refers to a malignant tumor.

By "metastasis" is meant the spread of cancer cells from its original site to another part of the body. The formation of metastasis is a very complex process and normally involves detachment of cancer cells from a primary tumor, entering the body circulation and settling down to grow within normal tissues elsewhere in the body. When tumor cells metastasize, the new tumor is called a secondary or metastatic tumor, and its cells normally resemble those in the original tumor. This means, for example, that, if breast cancer metastasizes to the lungs, the secondary tumor is made up of abnormal breast cells, not of abnormal lung cells. The tumor in the lung is then called metastatic breast cancer, not lung cancer.

According to the invention, "staging" of a cancer preferably refers to the classification of the progression and extent of a cancer. A preferred cancer staging system is the TNM classification of malignant tumors; wherein T describes the size of the tumor and whether it has invaded nearby tissue. N describes regional lymph nodes that are involved, and M describes distant metastasis. Each of these parameters is given a particular value depending on the situation in the patient, wherein generally a higher number indicates a more severe situation (T(0-4), N(0-3), M(0/1)). Additionally, for a more detailed classification further parameters can be determined and/or prefixes can be used. Furthermore, the TNM classification may be summarized in a cancer staging system according to the UICC, referring to cancer of from stage 0 to stage IV.

According to the invention, a "sample" in particular refers to but is not limited to a tissue sample, a body fluid and/or a cellular sample and may be obtained by conventional manners such as by tissue biopsy, including punch biopsy or by taking blood, bronchial aspirate, sputum, urine, feces or other body fluids or tissue sections, slides, etc. containing or suspected of containing cancer cells. According to the invention, the term "sample" also includes fractions or components of respective samples.

The terms "cell proliferation" and "to proliferate" as used herein refer to the amplification of the cell by cell division.

The term "cancer stem cells" in particular relates but is not limited to cells capable of generating aggregates of undifferentiated cells, so called tumor spheres, under suitable conditions *in vitro.* The cells that form spheres are capable of seff-renewal; when they are dissociated and grown under the same conditions, they will form spheres again. *In vivo,* cancer stem cells are characterized by their potential to form metastases and the expression of stem cell markers such as, e.g., CD44. They may also provide drug resistance. The terms "cancer stem cells" and "cancer initiating cells" are used as synonyms herein.

The term "tumor-associated carbohydrate antigen" in particular refers to a carbohydrate antigen that is expressed on cancer and/or tumor cells, in particular on malignant cancer and/or malignant tumor cells,

The term "tumor-specific carbohydrate antigen" in particular refers to a carbohydrate antigen that is predominantly or even exclusively expressed on cancer and/or tumor cells and thus not or only to a low extent on non-cancer respectively non-tumor cells. Preferably, the term "tumor-specifₗe carbohydrate antigen" refers to a carbohydrate antigen that is predominantly or preferably exclusively expressed on malignant cancer and/or malignant tumor cells and thus not or only to a low extent on non-cancer respectively non-tumor cells, on benign cancer and/or benign tumor cells and/or on healthy tissue of the same patient. Preferentially, the tumor-specific carbohydrate antigen is not expressed on most normal cells, even more preferred it is expressed only on few normal cells or cell types, even more preferred the expression on these normal cells has a special localization, e.g. strictly apical or in between the tight junctions, so that a binding molecule administered systemically, and especially i.v., can not or barely reach the antigen on these normal cells, even more preferred it is not expressed on normal epithelial cells. most preferred it is not expressed on normal cells.

"CD176" refers to the tumor-associated Thomsen-Friedenreich antigen (TF). TF exists in two forms, TFalpha and TFbeta, which can be linked to proteins or glycolipids. Core-1 is the disaccharide Galbetal-3Ga1NAc. which is O-glycosidically linked in an alpha-anomeric configuration in particular to the hydroxy amino acids serine or threonine or proteins in tumor cells. Core-1 corresponds to the TFalphe structure. While this disaccharide is a ubiquitous core structure found in a cryptic manner on many membrane glycoproteins of normal cells, its exposure and hence de novo occurrence of the antigen on tumor cells is restricted to a few specific carrier proteins. It has been demonstrated that CD176 is expressed on the surface of various cancer cells, such as breast carcinomas (lmai et al. 2001; Goletz et al. 2003), colorectal carcinomas (Cao et al. 1995), hepatocellular carcinomas (Cao et al. 1999), several leukemias (Cao et al. 2008) and other types of cancer. As a functional molecular entity, CD176 on the surface of cancer cells is involved in the invasive and metastatic properties of the cells (Cao et al. 1995). Hereinafter, the term CD176 preferably refers to the tumor-specific core-1 structure Gal*beta*1-3GaINAc*alpha*1-O-.

"CD175" refers to the Tn antigen, which is a tumor-associated carbohydrate antigen with the structure GaLNAcalpha1-O-. It is expressed on most epithelial cancers in a high percentage of cases and on several leukemias. To some extent it is also expressed on benign tumors. CD176 is a tumor-specific antigen.

"CD175s" refers to the s-Tn antigen (also referred to as sialyl-Tn or TAG-72), which is a tumor-associated carbohydrate antigen with the structure NeuAca/pha2-6Ga)NAc*alpha*1-O-. It is expressed on most epithelial cancers in a high percentage of cases and on several leukemias.

"CD173" refers to the blood group O antigen (also referred to as H type 2), which is a tumor-associated carbohydrate antigen with the structure Fuc*alpha*1-2Gal*beta*1-4GLcNA*cbeta*1-. It is expressed on red blood cells and endothelia of blood group O individuals. It is a cancer-related antigen in patients with blood groups other than O.

"CD174" refers to the Lewis Y antigen (also referred to as Le Y), which is a tumor-associated carbohydrate antigen with the structure Fuc*alpha*1-2Gal*beta*1-4[Fuc*alpha*1-3GlcNAc*beta*1-. CD174 is tumor-specific on many epithelial cancers, on several leukemias, and on haematopoietic progenitor cells.

"CA19-9" refers to the sialyl-Le a antigen, which is a tumor-associated carbohydrate antigen of the structure NeuAc*alpha*2-3Gal*beta*1-3[Fuc*alphal*-4]GlcNAc*beta*1-3(3a[*beta*1-, It is highly expressed on gastrointestinal and pancreatic carcinomas, but not on breast, kidney or prostate tumors.

"CD34", a membrane glycoprotein (105-120 kDa), is a well known marker of hematopoietic progenitor cells. It is also expressed on most leukaemia cells.

"CD44" is an adherence molecule (H-CAM, Pgp-1) of varying molecular weight. It is a cell surface hyaluronan receptor, interacts with matrix metalloproteinases, and plays a key role in cell migration. CD44 has been described as a cancer stem cell marker in breast, ovarian, pancreatic, prostate, colon, gastric, and other cancer types (see Li et al, 2007, and Takaishi et al, 2009). It is also a marker of normal pluripotent stem cells. On some cancer types splicing variants of CD44 occur such as CD44v6 on colorectal cancer.

"CD133" (prominin, 120 kDa) is a 5-transmembrane domain glycoprotein. The gene encodes an 865-amino acid glycoprotein and is conserved throughout the animal kingdom. It has been described as a cancer stem cell marker in many cancer types (e.g. colorectal, pancreatic, prostate. non-small cell lung, and hepatocellular carcinomas, as well as melanomas and glioblastomas)

"CD164" (MGC-24, 24 kDa) is a marker of hematopoietic progenitor cells, and also a marker of cancer stem cells in gastric carcinoma (see Masuzawa et al, 1992).

The terms "cell" and "cells" and "cell population" used interchangeably, in particular refers to one or more cells, in particular mammalian cells. The term includes progeny of a cell or cell population. Those skilled in the art will recognize that "cells" include progeny of a single cell, and the progeny can not necessarily be completely identical (in morphology or of total DNA complement) to the original parent cell due to natural, accidental. or deliberate mutation and/or change.

An "effective amount" is an amount sufficient to effect beneficial or desired clinical results. An effective amount can be administered in one or more administrations. An effective amount of the binding agent as active ingredient in particular is an amount that is sufficient to diagnose, palliate, ameliorate, stabilize, reverse, slow or delay the progression of the disease state. Toxicity and therapeutic efficacy of the active ingredient can be e.g. determined according to standard pharmaceutical procedures in cell cultures and/or experimental animals, including, for example, determining the LD₅₀ (the dose lethal to 50 percent of the population) and the ED₅₀ (the dose therapeutically effective in 50 percent of the population). The dose ratio between toxic and therapeutic effects is the therapeutic index and it can be expressed as the ratio LD₅₀ZED₅₀ Binding agents that exhibit large therapeutic indices are preferred. The data obtained from cell culture and/or animal studies can be used in formulating a range of dosages for humans. The dosage of the active ingredient typically lines within a range of circulating concentrations that include the ED₅₀ with low toxicity. The dosage can vary within this range depending upon the dosage form employed and the route of administration utilized.

### DETAILED DESCRIPTION OF THE INVENTION

The present invention is based on the finding that tumor-associated carbohydrate antigens are expressed on cancer stem cells and thus, are useful as cancer stem cell markers. This finding was surprising, as the cancer stem cell markers described so far are almost all proteins. Thus, the present invention pertains to the use of a tumor-associated, preferably tumor-specific carbohydrate antigen, preferably selected from CD176, CD175, CD175s. CD174. CD173 and CA19-9, more preferred from CD176, CD175 and CD175s, as cancer stem cell marker.

According to a first aspect, the invention provides a binding agent specifically binding a tumor-associated carbohydrate antigen for use in the treatment of cancer stem cells expressing said tumor-associated carbohydrate antigen.

The present inventors have shown that several tumor-associated carbohydrate antigens such as CD176, CD174 and CD173 are expressed on cancer stem cells and thus, are novel cancer stem cell markers. The identification of tumor-associated carbohydrate antigens as cancer stem cell markers provides novel therapeutic applications for agents specifically binding a tumor-associated carbohydrate antigen. Agents specifically binding a respective tumor-associated carbohydrate antigen can now be therapeutically used for targeting cancer stem cells that express said tumor-associated carbohydrate antigen. This provides the opportunity of therapeutic treatments that target and preferably kill cancer stem cells. The respective therapeutic agents can for example be used to target and thus destroy cancer stem cells which are resistant to regular chemotherapy. Thereby, improved cancer therapies are provided with the present invention.

According to one embodiment, the tumor-associated carbohydrate antigen is expressed predominantly or even exclusively on cancer stem cells. According to another embodiment, the tumor-associated carbohydrate antigen is expressed on the cancer stem cells as well as on cancer cells that are no cancer stem cells. If the tumor-associated carbohydrate antigen is expressed on both cell populations, this has the advantage that treatment with the binding agent specifically binding the tumor-associated carbohydrate antigen targets both cell populations. This is e.g. of importance in rare but documented cases where during tumor growth new cells with cancer stem cell properties develop, e.g. by epithelial-mesenchymal transition (EMT).

According to one embodiment, the binding agent specifically binding the tumor-associated carbohydrate antigen is therapeutically active. One example of a respective embodiment is the use of a therapeutically active antibody or antigen-binding fragment or derivative thereof as binding agent. According to a further embodiment, the binding agent specifically binding the tumor-associated carbohydrate antigen functions as a targeting molecule and is coupled to at least one therapeutic agent. Coupling can be achieved by covalent or non-covalent means. When the binding agent specifically binding the tumor-associated carbohydrate antigen functions as a targeting molecule, it can be itself therapeutically active or it may not be therapeutically active. In case it is not therapeutically active, it basically functions as a molecular carrier which brings the actual therapeutic agent (e.g. a radiopharmaceutical, chemotherapeutic agent or a toxin) to the desired target side of action, namely the cancer stem cells expressing the tumor-associated carbohydrate antigen. The therapeutic agent coupled to the binding agent specifically binding the tumor-associated carbohydrate antigen can be for example a chemotherapeutic agent or other anti-cancer drug

According to one embodiment, the tumor-associated carbohydrate antigen is selected from the group consisting of CD176, CD175, CD175s, CD174, CD173 and CA19-9. Preferably, the tumor-associated carbohydrate antigen is selected from CD176, CD173, CD174 and CD175 or CD175s. Details regarding these tumor-associated carbohydrate antigens are described above. It was shown by the inventors that these tumor-associated carbohydrate antigens are novel and suitable cancer stem cell markers.

Preferably, the tumor-associated carbohydrate antigen is tumor-specific. This has the advantage that the risk is reduced that non-tumor, respectively non-cancer cells are affected by the treatment. Details regarding the preferred tumor-specificity are also described above; it is referred to the respective disclosure.

According to one embodiment, cancer stem cells are treated with said binding agent which co-express at least one stem cell marker which, preferably, is a glycoprotein. The inventors have found that tumor-associated carbohydrate antigens such as in particular CD176, CD173 and CD174 are co-expressed with other stem cell markers such as CD44 and CD133, which are already known to be cancer stem cell markers. According to a preferred embodiment, cancer stem cells are treated which express at least one stem cell marker which is a (glyco)protein that carries the tumor-associated carbohydrate antigen that is specifically bound by the binding agent. The inventors have demonstrated that tumor-associated carbohydrate antigens useful as cancer stem cell markers such as CD176 are often almost exclusively carried on proteins which are known to be markers of normal as well as cancer stem cells, such as CD34, CD44. CD133, CD164 and/or are carried by tumor-associated (glyco)proteins. This suggests that normal (glyco)protein stem cell markers and/or tumor-associated (glyco)proteins undergo characteristic changes in their glycosylation during the process of malignant transformation to a cancer stem cell which result in the appearance (expression) of the tumor-associated carbohydrate antigen(s) identified as cancer stem cell markers. Thus, the identification of tumor-associated carbohydrate antigens as novel cancer stem cell markers provides a valuable therapeutic target for the treatment of cancer stem cells.

According to one embodiment, the cancer stem cells express one or more stem cell markers selected from the group consisting of CD34, CD44, CD44v6, CD133 and CD164. Preferably, said tumor-associated carbohydrate antigen is carried on a glycoprotein that is selected from the group consisting of CD34, CD44, CD44v6, CD133 and CD164. E.g. it was found that CD176, CD175 and CD175s are expressed *inter alia* on CD44 and CD133.

According to one embodiment, the cancer stem cells are of a cancer having one or more of the following characteristics:
a) It is a solid tumor;
b) It is a leukemia;
c) It is a multiple myeloma or lymphoma;
d) It is a tumor of epithelial origin; and/or
e) It is a tumor selected from the group consisting of lung, breast, liver, ovarian, gastrointestinal, pancreatic, prostate, cervical and head and neck cancer.

For the embodiment, wherein the cancer stem cells express CD176, CD175 or CD175s as tumor-associated carbohydrate antigen, the cancer stem cells preferably are of a leukemia or of a cancer which forms a solid tumor, i.e. it is a cancer other than leukemias and lymphomas. Preferably, the cancer is an epithelial malignancy and more preferred is selected from the group consisting of lung, breast and liver carcinoma. As is demonstrated in the examples provided herein, CD176 is expressed on cancer stem cells of these cancer types. Furthermore, it was found that CD176 is co-expressed with e.g. CD34 on leukemias.

For the embodiment, wherein the cancer stem cells express CA 19-9 as tumor-associated carbohydrate antigen, the cancer stem cells are according to one embodiment of a cancer which forms a solid tumor. Preferably, the cancer is a gastrointestinal carcinoma.

According to one embodiment, the binding agent specifically binds said tumor-associated carbohydrate antigen, which is a tumor-specific carbohydrate antigen. Preferably, under physiological conditions and thus conditions that occur during the therapeutic application of the binding agent, the binding agent does not bind an antigen, in particular a carbohydrate antigen, expressed on non-cancer cells, non-tumor cells, benign cancer cells and/or benign tumor cells. Using a respectively tumor-specific binding agent has several advantages. It ensures that when used in therapy the binding agent only recognizes and thus binds the target cells and thus cells that express the tumor-specific carbohydrate antigen but does not bind non-target, in particular benign or normal cells that express merely similar antigens, in particular similar carbohydrate antigens. This ensures a tumor, respectively cancer-specific therapy, thereby reducing the risk of unwanted side-effects. Suitable tumor-specific binding agents are described subsequently, in particular for the embodiment wherein the tumor-specific carbohydrate antigen is CD176 or CD175.

According to a preferred embodiment, the binding agent specifically binding a tumor-associated carbohydrate antigen is an antibody or an antigen-binding fragment or derivative of an antibody. Suitable examples are described above and in the examples.

The antibody or an antigen-binding fragment or derivative of an antibody used as binding agent according to the present invention preferably has one or more of the following characteristics:
a) it mediates ADCC and/or CDC of cancer and/or tumor cells, in particular cancer stem cells;
b) it induces and/or promotes apoptose of and/or tumor cancer cells, in particular cancer stem cells;
c) it inhibits proliferation of the target cancer and/or tumor cells, in particular cancer stem cells;
d) it induces and/or promotes phagocytosis of cancer and/or tumor cells, in particular cancer stem cells and/or
e) induces and/or promotes the release of cytotoxic agents.

According to one embodiment, a CD176 specific antibody or an antigen-binding fragment or derivative thereof is used for treating cancer stem cells expressing CD176. Preferably, said CD176 specific antibody or antigen-binding fragment or derivative thereof comprises at least one complementarity determining region (CDR) selected from the group consisting of CDRH1 having the amino acid sequence of SEQ ID No. 1, CDRH2 having the amino acid sequence of SEQ ID No. 2 or 3, and CDRH3 having the amino acid sequence of SEQ ID No. 4 or 5 or 6. More preferably, it comprises at least two CDRs, such as a CDRH1 and a CDRH2, a CDRH1 and a CDRH3, or a CORH2 and a CDRH3, each having the amino acid sequences as defined above. Most preferably, it comprises all three CDRS, that is a CDRH1, a CDRH2 and a CDRH3, each having the amino acid sequences as defined above. Alternatively, CDRH1 may have the amino acid sequence of any one of SEQ ID NOs: 14 to 17 and/or CDRH2 may have the amino acid sequence of any one of SEQ ID NOs: 18 to 27. In particular, these CDRs are present in a heavy chain variable region of the CD176 specific antibody or functionally active fragment or derivative thereof. The heavy chain variable region of the CD176 specific antibody or antigen-binding fragment or derivative thereof preferably contains or consists of the amino acid sequence of any one of SEQ ID Nos. 46 to 79.

Furthermore, said CD176 specific antibody or antigen-binding fragment or derivative thereof preferably comprises at least one complementarity determining region (CDR) selected from the group consisting of CDRL1 having the amino acid sequence of SEQ 11) No. 7 or 8 or 9, CDRL2 having the amino acid sequence of SEQ ID No. 10 or 11, and CDRL3 having the amino acid sequence of SEQ ID No. 12 or 13. More preferably, it comprises at least two CDRs, such as a CDRL1 and a CDRL2, a CDRL1 and CDRL3, or a CDRL2 and a CDRL3. each having the amino acid sequences as defined above. Most preferably, it comprises all three CDRs, that is a CORL1, a CDRL2 and a CDRL3, each having the amino acid sequences as defined above. Alternatively, CDRL1 may have the amino acid sequence of any one of SEQ ID NOs: 28 to 45. In particular, these CDRs are present in a light chain variable region of the CD176 specific antibody or antigen-binding fragment or derivative thereof. The light chain variable region of the CD176 specific antibody or antigen-binding fragment or derivative thereof preferably contains or consists of the amino acid sequence of any one of SEQ ID Nos. 80 to 94.

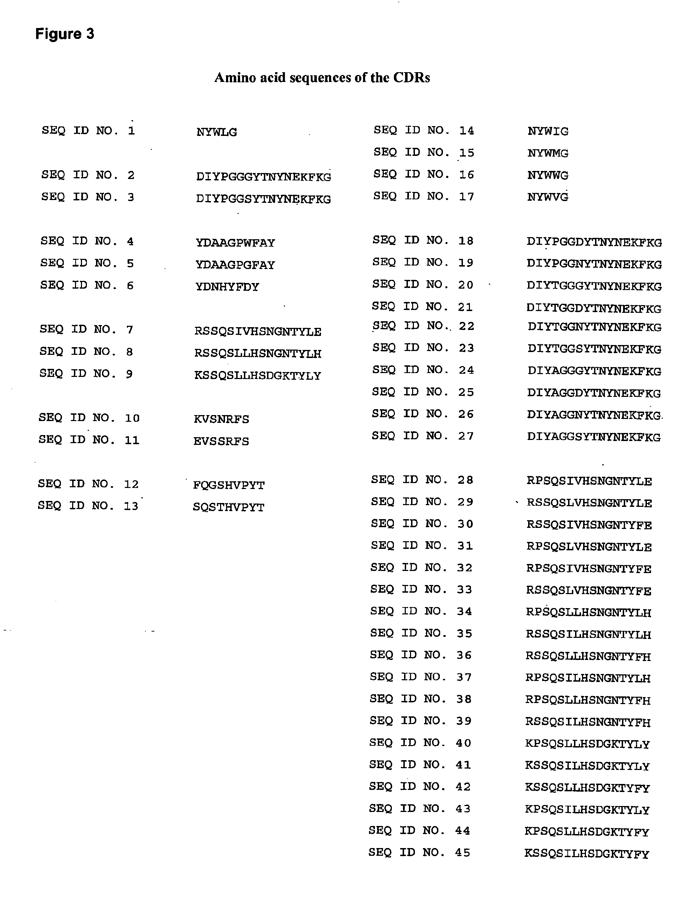

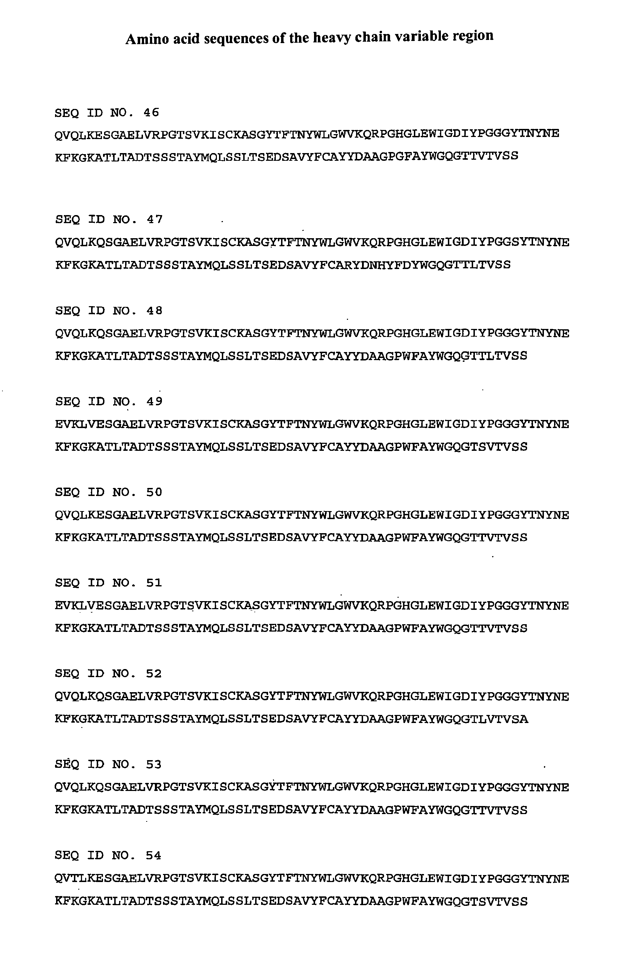

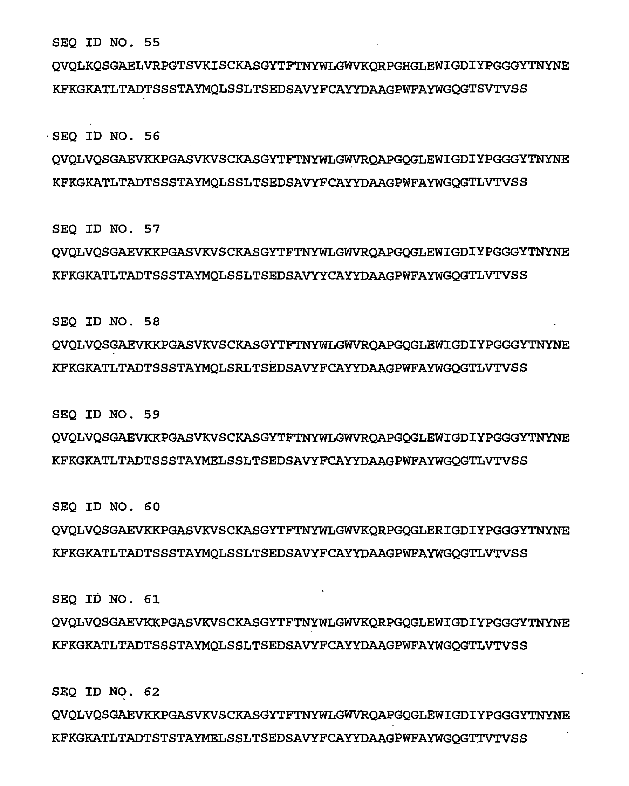

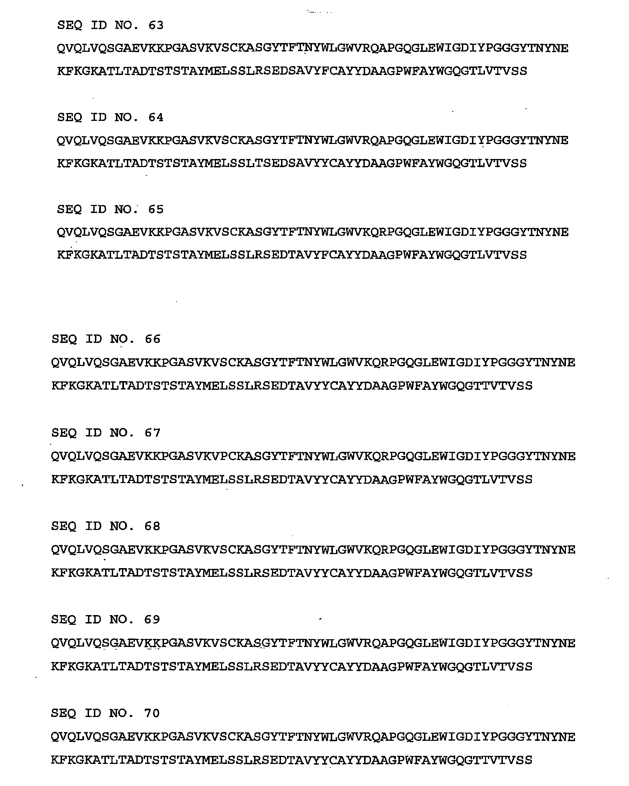

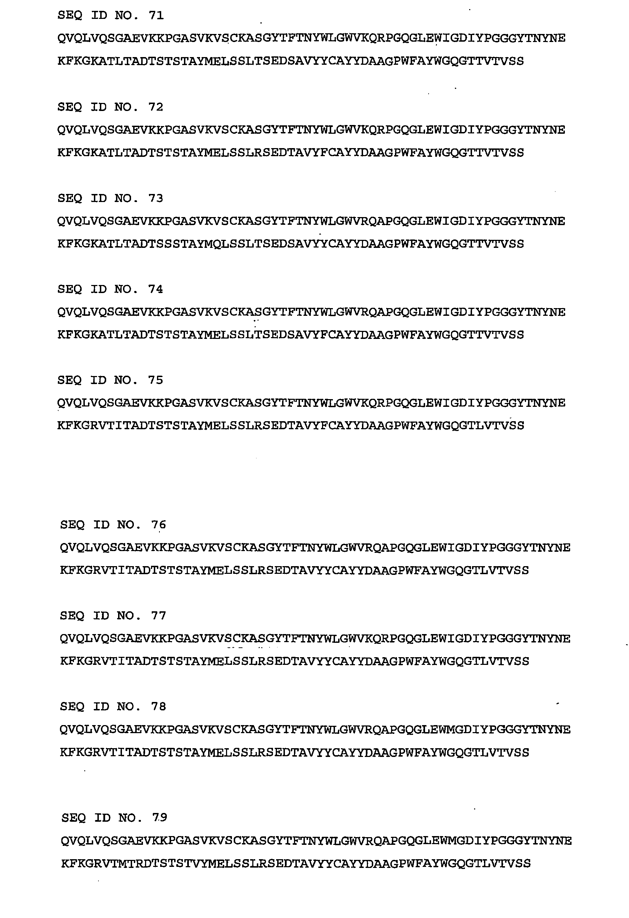

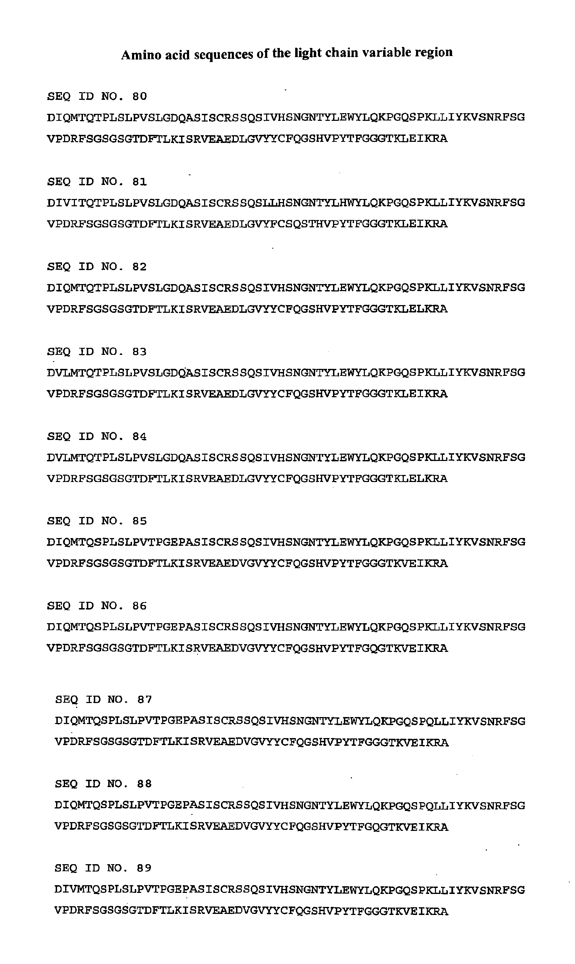

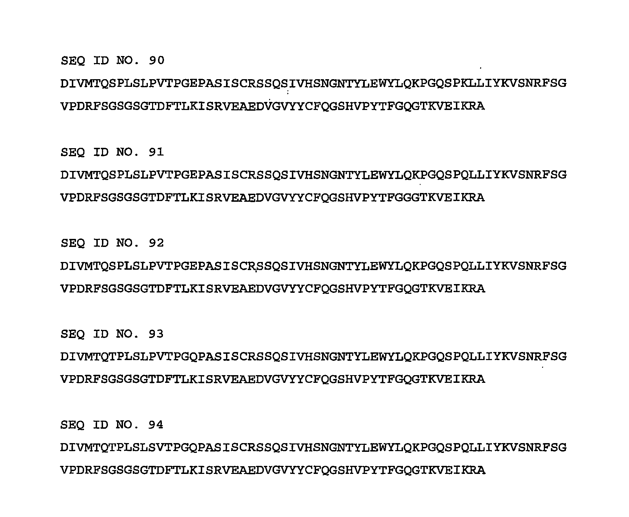

The sequences are shown in Figure 3. As the respective sequences are also disclosed in-EP 1572-747, the same numbering for the sequences is used herein.

The CD176 specific antibody or antigen-binding fragment or derivative thereof of the binding agent according to the invention may be a human, murine, humanized or chimeric antibody or a functionally active fragment or derivative thereof. Furthermore, it may be a single-chain antibody fragment (e.g. scFv), Fv fragment, Fab fragment, F(ab)₂ fragment, multibody (e.g. dia-, tria-, or tetrabody), an immunoglobulin of the IgG. IgM. IgA, IgE, IgD isotype or any subclass thereof, such as lgG1, or immunoglobulin-derived recognition molecules comprising at least one constant domain, or those lacking the (whole or part of the) J-chain.

In particularly preferred embodiments, the CD176 specific antibody or antigen-binding fragment or derivative thereof does not specifically interact with Gala1-3GalNAca, Galal-3GalNAcβ, GalNAca, Neu5Aca2-3Galβ1-3GalNAca, Galβ1-3(Neu5Aca2-6)GalNAca, GicNAcβ1-2Galβ1-3GaINAca, GtcNAca1-3Galβ1-3GalNAca. GalNAca1-3Galβ and/or 3'-O-Su-Galβ1-3GalNAca under physiological conditions such as are e.g. described in example 7 of EP 1 572 747.

According to one embodiment, the CD176 specific antibody binds the core-1 structure and shows a cross reactivity with the structure core-2, which is GlcNAcbeta1-6[Gal*beta*1-3]GalNAc*alpha*1-O-.

Examples of suitable antibodies include but are not limited to HH8 (Clausen H et al, Mol lmmunol 25:199-204 (1988)). A78-G/A7 (Glycotope GmbH, Berlin, Germany), Nemod-TF1 and Nemod-TF2 (Glycotope GmbH, Berlin, Germany) and HB-TF1 (DakoCytomation, Hamburg, Germany).

Furthermore, the binding agent according to the invention may comprise an antibody or an antigen-binding fragment or derivative thereof which shows a cross-reactivity with the CD176 specific antibody or antigen-binding fragment or derivative thereof described above. In particular, the binding agent according to the invention may be an antibody or an antigen-binding fragment or derivative thereof which binding can be effectively inhibited by a CD176 specific antibody or antigen-binding fragment or derivative thereof described above or by the CD176 structure in solution or coupled to a carrier molecules such as polyacrylamide or to a peptide, protein or lipid in sufficient concentrations or amounts determinable by those skilled in the art.

According to a further embodiment, a CD175 specific antibody or an antigen-binding fragment or derivative thereof is used for treating cancer stem cells expressing CD175. Suitable specific antibodies can be obtained by methods known in the prior art and/or described above. Known antibodies against CD175 are e.g. HB-Tn1 (obtainable from DakoCytomation, Hamburg, Germany), TKH6 (Kjeldsen et al, Cancer Res 48:2214, 1988), and 1E3 (Thumher et al, Glycobiology 4:429, 1994).

According to one embodiment, a CD175s specific antibody or an antigen-binding fragment or derivative thereof is used for treating cancer stem cells expressing CD175s. Suitable specific antibodies can be obtained by methods known in the prior art and/or described above. Known antibodies against CD175s are e.g. HB-STn1 (obtainable from DakoCytomation- Hamburg, Germany), TKH2 (Kjeldsen et al, Cancer Res 48:2214, 1988), B72.3 (Zhang et al, Cancer Res 55:3364. 1995), and MLS102 (Nakada et al, J Biol Chem 266:12402,1991).

According to one embodiment, a CD174 specific antibody or an antigen-binding fragment or derivative thereof is used for treating cancer stem cells expressing CD174. Suitable specific antibodies can be obtained by methods known in the prior art and/or described above. Known antibodies against CD174 are e.g. A70-C/C8 (Glycotope GmbH, Berlin, Germany), BR55-2 (Blasaczyk-Thurin et al, J Biol Chem 262:372, 1987), and NCC-ST-433 (Watanabe et al, Gann 76:43, 1985). Known therapeutic antibodies against CD174 are, e.g., ABL364 (Sandoz) and BR96 (Bristol-Myers Squibb).

According to one embodiment, a CD173 specific antibody or an antigen-binding fragment or derivative thereof is used for treating cancer stem cells expressing CD173. Suitable specific antibodies can be obtained by methods known in the prior art and/or described above, Known antibodies against CD173 are, e.g., A46-B/B10 (Glycotope GmbH, Berlin. Germany), BE2 (Young et al, J Biol Chem 256:10967,1981), and 92FRA2 (Dako Cytomation, Hamburg, Germany).

According to one embodiment, a CA19-9 specific antibody or an antigen-binding fragment or derivative thereof is used for treating cancer stem cells expressing CA19-9. Suitable specific antibodies can be obtained by methods known in the prior art and/or described above. Known antibodies against CA19-9 are, e.g., 1116NS-19-9 (Magnani et al, Cancer Res 43:5489, 1983), 121SLE (Herrero-Zabaleta et al, Bull Cancer 74:387, 1987).

Also provided is a pharmaceutical composition comprising a binding agent specifically binding a tumor-associated carbohydrate antigen for use in the treatment of cancer stem cells expressing said tumor-associated carbohydrate antigen(s), wherein the binding agent, the cancer stem cells and the tumor-associated carbohydrate antigen have one or more characteristics as are described above and in the claims. It is referred to the respective disclosure.

In another embodiment, a method for the treatment of cancer stem cells in a subject is disclosed, including administering to the subject, in an amount effective for the treatment, a pharmaceutical composition including (a) a binding agent specifically binding a tumor-associated carbohydrate antigen expressed on said cancer stem cells to be treated and (b) a pharmaceutically acceptable carrier. Details regarding the characteristics of the binding agents, the tumor-associated carbohydrate antigens as well as the cancer stem cells are described above in conjunction with the purpose bound compound claim. It is referred to the above disclosure. The subject methods are useful for both prophylactic and therapeutic purposes. As used herein, the term "treating" or "treatment" is used to refer to both prevention of disease, and treatment of a pre-existing condition. The treatment of ongoing disease, to stabilize or improve the clinical symptoms of the patient, is a particularly important benefit provided by the present invention. Such treatment is desirably performed prior to loss of function in the affected tissues; consequently, the prophylactic therapeutic benefits provided by the invention are also important. For example, treatment of a cancer patient may be reduction of tumor size, elimination of malignant cells, prevention of metastasis, the prevention of relapse in a patient who has been put into remission, reduction, partial or complete killing of disseminated cancer, in particular tumor cells or metastasizing cancer, in particular cells including those in circulation or those during evasion or invasion, a prolongation of survival and/or a prolongation of the time to tumor respectively cancer progression, The binding agent according to the present invention which specifically binds a tumor-associated carbohydrate antigen expressed on cancer stem cells and the pharmaceutical compositions comprising the same are useful and are described for said purposes.

Pharmaceutical compositions can include, depending on the formulation desired, pharmaceutically-acceptable, non-toxic carriers of diluents, which are defined as vehicles commonly used to formulate pharmaceutical compositions for animal or human administration. The diluent is selected so as not to affect the biological activity of the combination. Examples of such diluents are distilled water, buffered water, physiological saline, PBS, Ringer's solution, dextrose solution, and Hank's solution. In addition, the pharmaceutical composition or formulation can include other carriers, adjuvants, or non-toxic, nontherapeutic, nonimmunogenic stabilizers, excipients and the like. The compositions can also include additional substances to approximate physiological conditions, such as pH adjusting and buffering agents, toxicity adjusting agents, wetting agents and detergents.

The composition can also include any of a variety of stabilizing agents, such as an antioxidant for example. When the pharmaceutical composition includes a polypeptide, the polypeptide can be complexed with various well-known compounds that enhance the in vivo stability of the polypeptide, or otherwise enhance its pharmacological properties (e.g., increase the half-life of the polypeptide, reduce its toxicity, enhance solubility or uptake). Examples of such modifications or complexing agents include sulfate, gluconate, citrate and phosphate. The polypeptides of a composition can also be complexed with molecules that enhance their in vivo attributes. Such molecules include, for example, carbohydrates, polyamines, amino acids, other peptides, ions (e.g., sodium, potassium, calcium, magnesium, manganese), and lipids.

Further guidance regarding formulations that are suitable for various types of administration can be found in Remington's Pharmaceutical Sciences, Mace Publishing Company, Philadelphia, Pa., 17th ed. (1985). For a brief review of methods for drug delivery, see, Langer, Science 249:1527-1533 (1990).

The pharmaceutical compositions described herein can be administered in a variety of different ways. Examples include administering a composition containing a pharmaceutically acceptable carrier via oral, intranasal, rectal, topical, intraperitoneal. intravenous, intramuscular, subcutaneous, subdermal, transdermal, intrathecal, and intracranial methods.

According to a further aspect of the present invention, a method is provided for identifying a cancer comprising cancer stem cells that is susceptible to treatment with a binding agent that specifically binds a tumor-associated carbohydrate antigen wherein said treatment effects the cancer stem cells, comprising determining whether a lancer sample obtained from a patient comprise cancer stems cells that express the tumor-associated carbohydrate antigen the binding agent is specific for, wherein the presence of said tumor-associated carbohydrate antigen on cancer stem cells indicates that the cancer is susceptible to treatment with the binding agent that specifically binds a tumor-associated carbohydrate antigen and wherein said treatment also effects the cancer stem cells.

This method according to the present invention allows to test whether the cancer stem cells of a cancer are susceptible to treatment with a binding agent that specifically binds a tumor-associated carbohydrate antigen. The results of the method provide valuable diagnostic information to the physician. E.g. in case the cancer would comprise cancer stem cells which do not express said tumor-associated carbohydrate antigen, treatment with a binding agent specifically binding said tumor-associated carbohydrate antigen would not affect the cancer stem cells and accordingly, would be useless against the cancer stem cells. However, in case it is shown by said method that the cancer stem cells express said tumor-associated carbohydrate antigen the binding agent is specific for, chances are good that treatment with said binding agent will also target and accordingly affect the cancer stem cells. Thus, the method according to the present invention provides valuable aid to the physician for choosing the best therapy for the patient and to estimate whether a certain treatment will affect the cancer stem cells of a cancer.

According to a related diagnostic aspect, a method is provided for diagnosing, staging and/or prognosing cancer and/or monitoring the susceptibility to treatment, comprising the step of analyzing the expression of a tumor-associated carbohydrate antigen on cells in a sample isolated from a patient, wherein the presence of cells expressing the tumor-associated carbohydrate antigen indicates the presence of cancer stem cells in said sample.

The presence of cancer stem cells in a patient sample can be indicative of the stage of a cancer. In addition, detection of cancer stem cells can be used to monitor response to therapy and to aid in prognosis. The information obtained by the methods according to the present invention is useful in prognosis and diagnosis, including analysing the susceptibility to acceleration of the disease, the analysis by active monitoring of the disease wherein it is analysed whether the cancer progresses and e.g. needs treatment, the status of a disease state, the response to changes in the environment, such as the passage of time, the treatment with a chosen therapeutic agent in particular a binding agent as described above, or other modalities. By analysing whether cells contained in the sample express a tumor-associated carbohydrate antigen and accordingly, the sample comprises cancer stem cells, the cells can also be classified as to their ability to respond to therapeutic agents and treatments. Furthermore, the information derived is useful in determining and/or predicting the metastatic behavior of a cancer. The tumor-associated carbohydrate antigens identified as cancer stem cell markers, in particular CD176. CD175 and CD175s, are often present in high amounts on metastases. The presence of the herein described tumor-associated carbohydrate antigens of cancer stem cells and their binding by the binding agents of the invention are often connected with bad prognosis, especially when the tumor-associated carbohydrate antigen is tumor-specific and more specifically, when it is CD176. In case cancer stem cells are identified by the expression of the tumor-associated carbohydrate antigen marker, the patient has an increased risk of developing metastases.

Cancers can be staged according to the present invention by analysis of the presence of cancer stem cells which are identified based inter alia by the use of the tumor-associated carbohydrate antigen as marker target. Staging is useful for prognosis and treatment.

According to one aspect of the diagnostic methods according to the present invention, the binding agents according to the present invention which specifically bind a tumor-associated carbohydrate antigen expressed on cancer stem cells are used for *in vivo* diagnostic, in particular in vivo imaging. A respective method is also useful for diagnostic purposes. E.g. it can be determined, whether cancer cells expressing the tumor-associated carbohydrate antigen expressed on cancer stem cells can be identified and/or located in the patient. If this is the case, there is a risk that there are cancer stem cells. As is described above and below, it is preferred that a second stem cell marker is detected to confirm and/or determine the nature of the cancer stem cells. Furthermore, the response to therapy can be monitored as it can be determined e.g. whether the tumor size descreases or whether metases develop. Furthermore, a respective method is advantageous to identify the suitable dosage for a patient. According to one embodiment, the binding agent is labelled, e.g. being a radiopharmaceutical comprising a radionuclide. However, the binding agent may also be coupled to other agents/compounds such as e.g. a PET tracer that allow *in vivo* imaging. Suitable compounds are known in the prior art and thus, do not need further description here. Details regarding the binding agents, the tumor-associated carbohydrate antigens, further cancer stem cell markers and cancer types are described above and below and also apply to the in *vivo* imaging embodiment. It is referred to the respective disclosure.

Thus, the identification of tumor-associated carbohydrate antigens such as CD176, CD174 and CD173 as cancer stem cell markers and the described methods of the invention that are based on respectively make use of that finding, provide important and advantageous diagnostic and prognostic tools.

According to a preferred embodiment, the methods according to the present invention comprise analysing whether the cells co-express at least one second cancer stem cell marker. Thereby, the phenotype of a cancer stem cell can be further confirmed. Identifying the presence of cells co-expressing the tumor-associated carbohydrate antigen and at least one second cancer stem cell marker in the analysed sample indicates/confirms that the respective cells are cancer stem cells and e.g., that the analysed sample contains cancer stem cells or that cancer stem cells were detected by the in vivo diagnostic method. Suitable cancer stem cell markers that can be determined in addition to the tumor-associated carbohydrate antigen depend on the cancer type. According to a preferred embodiment, the co-expression of at least one additional glycoprotein cancer stem cell markers is tested, that is selected from the group consisting of CD34, CD44, CD44v6, CD133 and CD164.

According to one embodiment, the tumor-associated carbohydrate antigen is selected from the group consisting of CD176, CD175, CD175s, CD174, CD173 and CA19-9. Details regarding said tumor-associated carbohydrate antigens are described in detail above. It is referred to the above disclosure.

Preferably, the tumor-associated carbohydrate antigen is tumor-specific and preferably is expressed on malignant cells only. According to one embodiment, the tumor-associated carbohydrate antigen is expressed predominantly or exclusively on cancer stem cells. According to another embodiment, the tumor-associated carbohydrate antigen is expressed on the cancer stem cells as well as on cancer cells that are no cancer stem cells themselves but e.g. are derivatives of these cells.

According to one embodiment, the presence of cancer stem cells in a sample is determined by quantitating the cells having the phenotype of the cancer stem cells described herein and accordingly, express the tumor-associated carbohydrate antigen and preferably, a further cancer stem cell marker. The identification of a greater number of cancer stem cells in the sample is indicative of a more aggressive cancer phenotype According to one embodiment, the cells present in the sample are quantitated (quantified/quantitatively analyzed) as to the expression of the tumor-associated carbohydrate antigen and optionally, at least one further cancer stem cell marker. The "stem cell" character of the cells that are positive for the tumor-associated carbohydrate antigen may be further confirmed experimentally e.g. by determining the ability of the cells to self-renew and proliferate in culture, e.g. in forming spheres. Alternatively or additionally, the cells can be tested for tumorgenicity in an animal model.

In one embodiment of the invention, a sample from a cancer patient, e.g. a patient suffering or suspected from a cancer as described above, is stained with at least one agent specific for the tumor-associated carbohydrate antigen. In case co-expression of at least one further cancer stem cell marker is analysed, it is preferred that the sample is also stained with at least one agent specific for said further cancer stem cell marker.

The analysis of staining patterns may provide information on the quantity of cancer stem cells present in the sample. Furthermore, it may provide information on the relative distribution of the cancer stem cells, which distribution may allow to predict the tumorigenicity of the cancer and may also allow to prognose the progression of the cancer.

According to one embodiment, samples, containing or suspected of containing cancer cells, are stained with at least one agent specifically binding the tumor-associated, preferably tumor-specific carbohydrate antigen and thus the carbohydrate cancer stem cell marker and optionally at least one further agent specifically binding at least one second cancer stem cell marker, preferably CD44.

Samples to be analysed by the methods of the invention may be obtained from a variety of sources, particularly from a biopsy sample. Cells of such samples can be separated by centrifugation. elutriation, density gradient separation, apheresis, affinity selection, panning, FACS, centrifugation with Hypaque, etc. prior to analysis. Once a sample is obtained, it can be used directly, frozen, or maintained in appropriate culture medium for short periods of time, or fixed in a suited fixation solution, or fixed and embedded in a medium suited for histologigal or immunohistological examination. Various media can be employed to maintain cells. The samples may be obtained by any convenient procedure, such as biopsy, or from surgical specimen. Usually a sample will comprise at least about 10² cells, more usually at least about 10³ cells, and preferable 10⁴, 10⁵ or more cells. Typically the samples will be from human patients, although animal models may find use, e.g. equine, bovine, porcine, canine, feline, rodent, e.g. mice, rats, hamster, primate, etc.

The samples may be frozen, embedded, fixed, present in a tissue microarray, and the like. The agents used for staining the tumor-associated carbohydrate antigen and, optionally, a further cancer stem cell marker can be e.g. binding agents specifically binding the cancer stem cell markers such as e.g. antibodies. Suitable examples are described above. These agents may be detectably labelled, or may be indirectly labelled in the staining procedure. According to one embodiment, the label can also be used for separating the tumor-associated carbohydrate antigen positive cells. Suitable labels as well as staining procedures are known in the prior art and accordingly, do not need further description here even though some examples are described herein. A standard procedure for analyis may include a histological fixation of the sample (e.g. by formalin) and subsequent staining as is e.g. described in the examples. The obtained data allows to determine the number and distribution of cancer stem cells in the sample.

Methods suitable for detecting and/or quantifying cells expressing the tumor-associated carbohydrate antigen include, for example, immunologic assays such ELISA, RIA, Western blot and immunohistochemistry, flow cytometry, immunohistochemistry or the like. These methods allow to identify the presence and if desired also the quantity of cells that express the tumor-associated carbohydrate antigen such as in particular CD176. CD175, CD175s, CD174 and CD173 as novel cancer stem cell markers and, optionally, at least one further cancer stem cell marker.

Of particular interest is the use of antibodies as binding agent which specifically bind the tumor-associated carbohydrate antigen. Conveniently, these antibodies are conjugated with a label for detection and/or for use in separation. Labels include magnetic beads, which allow for direct separation, biotin, which can be separated with avidin or streptavidin bound to a support, fluorochromes, which can be used with a fluorescence activated cell sorter, or the like, to allow for ease of separation of the particular cell type if desired. Fluorochromes that find use include phycobiliproteins, e.g. phycoerythrin and allophycocyanins, fluorescein derivatives, Cy3, Cy5, or Texas red. In case a further cancer stem cell marker is detected in addition to the tumor-associated carbohydrate antigen, it is preferred that the agent specifically binding said further cancer stem cell marker is labeled with a different fluorochrome, to permit independent sorting for each cancer stem cell marker.

According to one embodiment, the binding agents specifically binding the cancer stem cell markers) are added to a suspension of cells, and incubated for a period of time sufficient to bind the available cell surface antigens. It is desirable to have a sufficient concentration of agents in the reaction mixture, such that the efficiency of the separation is not limited by lack of agent specifically binding the cancer stem cell marker. The appropriate concentration is determined by titration. The medium in which the cells are separated will be any medium that maintains the viability of the cells, The labelled cells are then preferably quantitated as to the expression of the cell surface markers as previously described; a number of such methods are also-known in the art.

The comparison of a differential progenitor analysis; or a cancer stem cell analysis obtained from a patient sample, and a reference analysis may be accomplished by the use of suitable deduction protocols, artificial intelligence (Al) systems, statistical comparisons, etc. A comparison with a reference differential progenitor analysis from normal cells, cells from similarly diseased tissue, and the like, can provide an indication of the disease staging. A database of reference differential progenitor analyses can be compiled. An analysis of particular interest tracks a patient, such that acceleration of disease is observed at an early stage. The methods of the invention provide detection of acceleration prior to onset of clinical symptoms, and therefore allow early therapeutic intervention, e.g. initiation of chemotherapy, increase of chemotherapy dose, changing selection of chemotherapeutic drug or other anti-cancer drug, and the like.

Various methods can be utilized for quantifying the presence of the selected markers. For measuring the amount of a molecule that is present, a convenient method is to label a molecule with a detectable moiety, which may be fluorescent, luminescent, radioactive, enzymatically active, etc., particularly a molecule specific for binding to the parameter with high affinity. Fluorescent moieties are readily available for labelling virtually any biomolecule, structure, or cell type, immunofluorescent moieties can be directed to bind not only to specific proteins but also specific conformations, cleavage products, or site modifications like phosphorylation. Individual peptides and proteins can be engineered to autofluorescence, e.g. by expressing them as green fluorescent protein chimeras inside cells (for a review see Jones et al. (1999) Trends Biotechnol. 17(12):477-81). Thus, antibodies can be genetically modified to provide a fluorescent dye as part of their structure. Depending upon the label chosen, parameters may be measured using other than fluorescent labels, using such immunoassay techniques as radioimmunoassay (RIA) or enzyme linked immunosorbance assay (ELISA), homogeneous enzyme immunoassays, and related non-enzymatic techniques.

The identification of cells in the analysed sample which express the tumor-associated carbohydrate antigen and, optionally, at least one further cancer stem cell marker, indicates the presence of cancer stem cells in the analysed sample, and may also allow the definition of cancer stem cell domains in a primary tumor, as well as in metastases. Furthermore, as is described above, identifying cancer stem cells that express a specific tumor-associated carbohydrate antigen allows the selection of an appropriate therapy to treat the cancer including the cancer stem cells.

According to one embodiment, the binding agent used in the methods according to the present invention specifically binds a tumor-specific carbohydrate antigen. Preferably, the binding agent does not bind an antigen, in particular a carbohydrate antigen, expressed on non-cancer cells. non-tumor cells, benign cancer cells and/or benign tumor cells under the conditions used for performing the diagnostic related methods of the present invention described above and defined in claims 9 and 10. Using a respectively tumor-specific binding agent in the methods of the present invention has several advantages. It ensures that the binding agent only recognizes and thus binds the target cells and thus cells that express the tumor-specific carbohydrate antigen but does not bind non-target cells that express merely similar antigens, in particular similar coarbohydrate-antigens. This reduces the risk of false positives in the methods according to the present invention. Suitable tumor-specific binding agents are described above, in particular for the embodiment wherein the tumor-specific carbohydrate antigen is CD176.

According to a preferred embodiment, the binding agent specifically binding the tumor-associated carbohydrate antigen is an antibody or an antigen-binding fragment or derivative of an antibody.

According to one embodiment, an antibody or an antigen-binding fragment or derivative thereof specific for the tumor-associated carbohydrate antigen to be detected is used for staining cancer stem cells expressing said tumor-associated carbohydrate antigen. Suitable examples of antibodies binding CD176, CD175, CD175s, CD174, CD173 and CA19-9 are described in detail above. It is referred to the above disclosure.

Cancer types which express tumor-associated carbohydrate antigens and in particular express CD176, CD175, CD175s, CD174, CD173 and/or CA19-9 are described above. It is referred to the above disclosure.

In one embodiment, the patient sample is compared to a reference or a standard test value, In another embodiment, the patient sample is compared to a pre-cancerous sample, or to one or more time points through the course of the disease.

In some embodiments of the invention, methods are provided for classification and/or clinical staging of cancers according to the cancer stem cells that are present, wherein greater numbers of cancer stem cells are indicative of a more aggressive cancer phenotype.

In another embodiment of the invention, compositions of isolated cancer stem cells are provided which express a tumor-associated carbohydrate antigen. Thus, also provided is a composition of mammalian cancer stem cells, wherein at least 50%, preferably at least 75%, most preferred at least 90% of the cells in the composition are cancer stem cells that are positive for a tumor-associated. preferably tumor-specific carbohydrate antigen.

These cells respectively compositions are e.g. useful for experimental evaluation and as a source of lineage and cell specific products and as targets for the discovery of factors or agents that can affect them. These cancer stem cells respectively compositions may be used, for example, in a method of screening an agent for an effect on the cancer stem cells. This involves combining the candidate agent with the cell population of the invention, and then determining any modulatory effect resulting from the agent. This may include examination of the cells for toxicity, metabolic change, or an effect on cell function. The phenotype of cancer stem cells described herein provides a means of predicting disease progression, relapse, and development of drug resistance.

Furthermore, said compositions of isolated cancer stem cells which express a tumor-associated carbohydrate antigen or lysates, fragments or fractions thereof comprising the tumor-associated carbohydrate antigen are useful for immunotherapy purposes for targeting cancer-stem cells. E.g. they may be used as autologous or allogenic vaccines. These cancer stem cell compositions, as whole, living or dead (preferably, the cells can not divide), as a lysate, fragment, fraction or purification of these alone or in combination with other agents such as adjuvants can be used to immunize either animals or humans in order to achieve an immune response against the cancer stem cells, a cancer expressing the tumor-associated carbohydrate antigen, a metastasis expressing the tumor-associtaed carbohydrate antigen and/or a dissiminating tumor cell expressing the tumor-associtaed carbohydrate antigen. Preferably, they induce an immune response against the tumor-associated carbohydrate itself. Preferably, at least one binding agent against the cancer stem cell expressing a tumor-associated, preferably a tumor-specific carbohydrate antigen is thereby obtained. Preferably, the obtained binding agent is tumor-specific and thus only recognises and binds the tumor-specific carbohydrate antigen but no similar carbohydrate structure that is not expressed on cancer cells, preferably cancer stem cells.

According to one embodiment, the cancer stem cells or lysates, fragments or fractions thereof comprising the tumor-associated carbohydrate antigen are used for obtaining antigen presenting cell based vaccines. Preferably, antigen presenting cells such as e.g. dendritic cell precursors are stimulated ex vivo to mature and to take up and present the tumor-associated carbohydrate antigen expressed on cancer stem cells. For this purpose, the antigen presenting cells can be co-incubated with the compositions according to the present invention and/or lysates, fragments or fractions thereof expressing a tumor-associated carbohydrate antigen. They may come from the patient who will receive the vaccine, or they may come from other patients with the same type of cancer. The mature dendritic cells can then by injected to the patient to elict an immune response against cancer stem cells that express the tumor-associated carbohydrate antigen. Suitable and preferred tumor-associated carbohydrate antigens are described above, it is referred to the respective disclosure.

The above described vaccines that are obtained by using the composition according to the present invention or lysates, fragments or fractions thereof which comprise the tumor-associated carbohydrate antigen expressed on cancer stem cells preferably induce an immune response against the cancer stem cells, preferably against the cancer stem cells of a solid tumor which prevents, reduces or cures the cancer, metastasis or dissiminating tumor cell growth.

Furthermore, said composition according to the present invention or lysates or fractions thereof comprising the tumor-associated carbohydrate antigen, are useful for identifying and/or isolating suitable binding agents useful for the therapeutic purposes described above. E.g. they can be used as screening targets for identifying suitable binding molecules that target cancer stem cells. Suitable screening methods are known in the prior art. According to one embodiment, cells, phages, bacteria or yeast, expressing and/or displaying binding agents are selected according to their binding to these aforementioned compositions according to the present invention, cancer stem cells expressing the tumor-associated carbohydrate antigen or to lysates, fractions or purifications thereof comprising the tumor-associated carbohydrate antigen, by screening technologies known to those skilled in the art. Furthermore, said composition according to the present invention or lysates or fractions thereof comprising the tumor-associated carbohydrate antigen are useful for obtaining suitable binding agents when used as an immunogen. Respective immunisation methods e.g. for obtaining suitable antibodies are known in the prior art and are also described above.

The cancer stem cells of interest expressing the tumor-associated carbohydrate antigen may be separated from a complex mixture of cells by techniques that enrich for cells having the above described characteristics. Examples are described above and include but are not limited to FACS sorting procedures or methods involving the use of magnetic particles which carry a binding agent that specifically binds said tumor-associated carbohydrate antigen. Suitable binding agents specifically binding the tumor-associated carbohydrate antigen are described above: it is referred to the respective disclosure.

According to one embodiment, the tumor-associated carbohydrate antigen is selected from the group consisting of CD176, CD175, CD175s. CD174, CD173, and CA19-9, and preferably is selected from CD176, CD174 and CD173. Details regarding said tumor-associated carbohydrate antigens are described in detail above. It is referred to the above disclosure.

Preferably, the composition comprises cancer stem cells which are positive for at least one further stem cell marker. Preferably, the cancer stem cells express a glycoprotein which is more preferred selected from the group consisting of CD34, CD44, CD44v6, CD133 and CD164.

Also provided is a method for the isolation of cancer stem cells, comprising the isolation of cancer stem cells which express the tumor-associated carbohydrate antigen, which preferably is selected from the group consisting of CD176, CD175, CD175s, CD174, CD173, and CA19-9, and more preferred is selected from CD176, CD174 and CD173. Said method comprises the following steps:
a) contacting a composition comprising or suspected of comprising cancer stem cells expressing a tumor-associated carbohydrate antigen with a binding agent specifically binding said tumor-associated carbohydrate antigen for binding cancer stem cells expressing a tumor-associated carbohydrate antigen.
b) separating at least one cancer stem cell expressing a tumor-associated carbohydrate antigen from the remaining composition.

Suitable binding agents, cancer types which express the respective tumor-associated carbohydrate antigens and other details are described above and below. It is referred to the above disclosure.

For isolation of respective cells from tissue, an appropriate solution may be used for dispersion or suspension. Such solution will generally be a balanced salt solution, e.g. normal saline, PBS, Hank's balanced salt solution, etc., conveniently supplemented with fetal calf serum or other naturally occurring factors, in conjunction with an acceptable buffer at low concentration, generally from 5-25 mM. Convenient buffers include HEPES, phosphate buffers, lactate buffers, etc.

The separated cells may be collected in any appropriate medium that maintains the viability of the cells, usually having a cushion of serum at the bottom of the collection tube. Various media are commercially available and may be used according to the nature of the cells, including dMEM, HBSS, dPBS, RPMI, Iscove's medium, etc., frequently supplemented with fetal calf serum.

Compositions highly enriched for cancer stem cells expressing a tumor-associated carbohydrate antigen are achieved in this manner. The subject population may be at or about 60 percent or more of the cell composition, and preferably be at or about 75 percent or more of the cell composition, and may be 90 percent or more. The desired cells are identified by their surface phenotype. Details are described above, it is referred to the respective disclosure. Preferably, their cancer stem cell phenotype is additionally confirmed by their ability to self-renew which is an essential property of stem cells. The enriched cancer stem cell population may be used immediately, or may be frozen at liquid nitrogen temperatures and stored for long periods of time, being thawed and capable of being reused. The cells will may be stored e.g. in 10 percent DMSO, 50 percent FCS, 40 percent RPMI 1640 medium. The population of cells enriched for cancer stem cells expressing the tumor-associated carbohydrate antigen and optionally a further cancer stem cell marker may be used in a variety of screening assays and cultures, as described below.

The enriched cancer stem cell population may be grown in vitro under various culture conditions. Culture medium may be liquid or semi-solid, e.g. containing agar, methylcellulose, etc. The cell population may be conveniently suspended in an appropriate nutrient medium, such as lscove's modified DMEM or RPMt-1640, normally supplemented with fetal calf serum (about 5-10 percent), L-glutamine, a thiol, particularly 2-mercaptoethanol, and antibiotics, e.g. penicillin and streptomycin.

The culture may contain growth factors to which the cells are responsive. Growth factors, as defined herein, are molecules capable of promoting survival, growth and/or differentiation of cells, either in culture or in the intact tissue, through specific effects on a transmembrane receptor. Growth factors include polypeptides and non-polypeptide factors. A wide variety of growth factors may be used in culturing the cells, e.g. LIF, steel factor (c-kit ligand), EGF, insulin, IGF, NGF, etc. In addition to, or instead of growth factors, the subject cells may be grown in a co-culture with fibroblasts, stromal or other feeder layer cells.

Also provided is a kit for diagnosing, staging and/or prognosing cancer, the metastatic behavior of cancer, in vivo imaging and/or monitoring the efficacy of a therapeutic cancer treatment, comprising a binding agent which specifically binds a tumor-associated carbohydrate antigen and instructions for use. Preferably, said binding agent binds a tumor-associated carbohydrate antigen expressed on cancer stem cells selected from the group consisting of CD176, CD175, CD175s, CD174, CD173, and CA19-9, and more preferred selected from CD176, CD174 and CD173.

Preferably, said binding agent is labelled. Suitable binding agents and labels are described above, it is referred to the respective disclosure. Kits may also include tubes, buffers, etc., and instructions for use.

According to a preferred embodiment, the kit comprises at least one further agent specifically binding a further stem cell marker. Preferably, said stem cell marker is a glycoproteins. According to a preferred embodiment, the kit comprises at least one further agents specifically binding a glycoproteins selected from the group consisting of CD34, CD44, CD44v6, CD133 and CD164.

Also provided is method of screening a candidate therapeutic agent for effectiveness against a cancer stem cell expressing a tumor-associated carbohydrate antigen, the method comprising:
a) Contacting said agent with the cell composition according to the present invention, and
b) Determining the effectiveness of said agent against said tumor-associated carbohydrate antigen.

The respective screening method is particularly useful for in vitro assays and screening methods to detect, identify and/or isolate factors and therapeutic agents, such as chemotherapeutic agents, binding agents or other anti-cancer drugs, that are active on cancer stem cells. Of particular interest are screening assays for agents that are active on human cells. A wide variety of assays may be used for this purpose, including immunoassays for protein binding, determination of cell growth, differentiation and functional activity, production of factors; and the like.

In screening assays for candidate therapeutic agents usually a culture comprising cancer stem cells expressing the tumor-associated carbohydrate antigen of interest is contacted with the binding agent of interest, and the effect of the agent assessed by monitoring output parameters, such as expression of markers, cell viability, and the like. The screening may also involve determining modulation of growth, proliferation, viability, and/or differentiation status of the cell in the presence of the candidate therapeutic agent as compared to the growth, proliferation, viability, and/or differentiation status of the cell in the absence of the candidate therapeutic agent.

Preferably, said tumor-associated carbohydrate antigen selected from the group consisting of CD176, CD175, CD175s, CD174, CD173. and CA19-9. and more preferred selected from CD176, CD174 and CD173. Details are described above, it is referred to the respective disclosure.

The cells may be freshly isolated, cultured, genetically altered as described above to provide a marker for activation of signaling pathways, and the like, The cells may be environmentally induced variants of clonal cultures: e.g. split into independent cultures and grown under distinct conditions, for example with or without drugs; in the presence or absence of cytokines or combinations thereof. The manner in which cells respond to an agent, particularly a pharmacologic agent, including the timing of responses, is an important reflection of the physiologic state of the cell.

Parameters are quantifiable components of cells, particularly components that can be accurately measured, desirably in a high throughput system.

Candidate agents are screened for biological activity by adding the agent to at least one composition of mammalian cancer stem cells, wherein at least 50% of the cells in the composition are positive for the tumor-associated carbohydrate antigen of interest. Further-characteristics of the composition-are - described above, we refer to the respective disclosure. The change in parameters in response to the agent is measured, and the result evaluated by comparison to reference cultures, e.g. in the presence and absence of the agent. obtained with other agents, etc.

The agents can be conveniently added in solution, or readily soluble form, to the medium of cells in culture. The agents may be added in a flow-through system, as a stream, intermittent or continuous, or alternatively, adding a bolus of the compound, singly or incrementally, to an otherwise static solution. In a flow-through system, two fluids are used, where one is a physiologically neutral solution, and the other is the same solution with the test compound added. The first fluid is passed over the cells, followed by the second. In a single solution method, a bolus of the test compound is added to the volume of medium surrounding the cells. The overall concentrations of the components of the culture medium should not change significantly with the addition of the bolus, or between the two solutions in a flow through method.

It is to be understood that this invention is not limited to the particular methodology, protocols, cell lines, animal species or genera, and reagents described, as such may vary. It is also to be understood that the terminology used herein is for the purpose of describing particular embodiments only, and is not intended to limit the scope of the present invention, which will be limited only by the appended claims.

### FIGURES

The figures show the results obtained with the examples described below.

### Figure 1.1

a-c: Confocal microscopy analysis performed with the lung cancer cell line NCIH446 (magnification x400). Cells were stained with monoclonal antibodies specific for CD44 (red) and CD176 (green). Nuclei were counterstained with DAPI (blue). CD44 is strongly expressed in most NClH446 cells (a). CD176 expression could be seen in the membrane of cell clusters (b). The mixed picture (c) demonstrates co-tocatization of CD44 and CD176 in these cell clusters (yellow).

**d-i:** Co-expression of CD44 and CD176 as well as of CD133 and CD176 in HCC tissues is shown by double immunofluorescence staining (magnification x200). The co-expression of CD176 (green, d)/CD44 (red, e) and CD133 (red, g)/CD176 (green, h) by overlay was found (yellow, f and i) respectively.

**j. k:** Expression of CD176 in lung cancer tissues as analyzed by immunohistochemistry (magnification x200). CD176 is found at the cellular surface and in the cytoplasm of lung adenocarcinoma cells (j) and of lung squamous carcinoma cells (k).

### Figure 1.2 A

FACS analysis of co-expression of CD44 and CD176 in cells of the cell lines SPC-A-1, GLC (lung adenocarcinoma) and HepG2 (HCC). Cells were incubated with anti-CD44 (lgG) and anti-CD176 (tgM), followed by incubation with anti-IgG-Cy3 (V-chain specific) and anti-IgM-FITC (u-chain specific). A minimum of 10,000 events were collected per sample. The percentage of cells with the respective combination of markers is indicated in each section of the graph, demonstrating a strong correlation of staining of both markers. Data shown are representative of several independent experiments.

### Figure 1.2B

FACS analysis of co-expression of CD44 and CD176 before and after 4-OHT treatment of MDA-435 (breast carcinoma) cells. The percentage of cells with the respective combination of markers is indicated in each section of the graph. The number of CD44⁺/CD176⁺ cells is increased after treatment with 4-OHT for 24h. Data shown are representative of several independent experiments,

### Figure 2.1:

Double immunofluorescence staining of breast cancer cells.

**a-c:** cell line MCF-7: d-l breast cancer tissue sections. Cells were stained with monoclonal antibodies G44-26 (CD44, IgG2b) or ANC9C5 (CD133), and with A'l0-CICB (CD174, ]gM) or A46-8/810 (CD173, IgM), followed by incubation with anti-IgG-Cy3 (y chain-specific, red) and anti-IgM-FITC (µ chain-specific, green).

**c, f, i, l** are merged pictures; nuclei are counterstained with DAPI (blue).

CD 173 (a,d) is in many cases co-expressed with CD44 (b, e). CD174 (g, j) shows co-expression with CD44 (h) as well as with CD133 (k). Magnification: 200x.

### Figure 2.2:

**A:** Flow-cytometric analysis of CD44⁺ICD173⁺ expression in the breast adenocarcinoma cell lines MDA-MB-435 and MCF-7. Cancer cells were incubated with anti-CD44 (IgG) and anti-CD174 (IgM) antibodies, respectively, followed by incubation with anti-IgG-Cy3 (y chain-specific) and anti-IgM-FITC (µ chain-specific). Values are taken from one of three similar experiments. Large proportions of both cell lines are positive for both markers (CD44 and H2).

**B:** Flow cytometric analysis of CD44'ICD174` expression on MDA-MB-231 cells before and after 4-OHT treatment. 4-OHT treatment results in an increase in the proportion of cells expressing both CD44 and CD174 (LeY). Values are taken from one of three similar experiments.

### Figure 2.3:

lmmunohistochemistry of an intraductal carcinoma section stained with the CD173 mAb A46-B/B₁0. Basal cells (stem cell-like cells) of the remaining duct walls are strongly stained.

### Figure 2.4:

Immunoprecipitation of lysates from breast adenocarcinoma cell lines MDA-MB-231, MDA-MB-435, and MCF. The CD₄₄-immunoprecipitated material was resolved by SDS-PAGE and .analyzed by immunoblotting using mAb CD173. The data show that in breast carcinomas CD173 (H2) is carrying CD173.

### EXAMPLES

### I Expression of CD176 (Thomson-Friedenrelch antigen) on lung, breast and liver cancer stem cells

### 1. Materials and methods

### Antibodies

Antibodies applied were: CD44 mAb (G44-26, BD Biosciences, Franklin Lakes, NJ, USA), CD133 mAb (ANC9C5, Ancell, Bayport, MN, USA, CD176 mAb (NM-TF2, Glycotope GmbH, Berlin, Germany). MUC1 mAb (PankoMab, Glycotope GmbH).

### Cell lines and cell culture

A broad variety of human cancer cell lines, including those from breast adenocarcinoma (MDA231, MDA435, and MCF-7), lung cancer (SPC-A-1 and GLC-82, lung adenocarcinoma; NCIH446, small cell lung carcinoma: 801-D. giant cell lung carcinoma), and hepatocellular carcinoma (HepG2 and HuH-7) were used in this study. All cell lines were routinely cultured in Dulbecco's Modified Eagle Medium (DMEM) containing 10% fetal calf serum.

### Immunocytochemistry

The cultured cells were plated onto polylysine (Sigma, Saint Louis, MO, USA)-coated slides in DMEM/F12 medium containing 10% fetal calf serum overnight. Thereafter, the medium was carefully aspirated, and the slides were air-dried. Wrapped slides could be stored at -80n until use. For immunofluorescence double staining cells were fixed with cold (-20°) acetone for 15 min, blocked with 2% bovine serum albumin (BSA) in phosphate-buffered saline (PBS for 30 min, incubated with antibodies against CD44 or CD133 together with mAb against CD176 for 60 min. The slides were subsequently incubated with a mixture of fluorescein isothiocyanate (FITC)-conjugated anti-mouse IgM (µ-chain specific) (F9259. Sigma) and Cy3-conjugated goat anti-mouse IgG (y-chain specific) (#69732, Jackson Laboratories. West Grove, PA, USA). Counterstaining was performed with 4',6-diamidino-2-phenylindole dihydrochloride (DAPI) (Beyotime Biotechnology, Jiangsu, China). Negative controls were performed with medium instead of the specific mAb. The slides were mounted with glycerol and analysed by confocal microscopy (Olympus, Tokyo, Japan) and fluorescence microscopy. The percentage of positive cells or double positive cells was counted on digital images.

### Flow cytometry analysis

Cell suspensions of cell lines were prepared at 1 x 10⁶ cells/100µl. Cells were washed twice with PBS containing 2% BSA and incubated with primary antibodies at appropriate dilutions at 4 ºC for 20 min, followed by anti-IgG-Cy3 (γ chain-specific) and anti-IgM-FITC (µ chain-specific) at appropriate concentrations at 4 °C for 20 min. Flow cytometry was performed with a FACScan (BD Biosciences. Franklin Lakes, NJ, USA). Collected data from 10,000 cells and WinMDl software were used in the analysis of FCS datafiles.

For 4-hydroxytamoxifen treatment, MDA231, MDA435 and MCF-7 breast adenocarcinoma cell lines were exposed to 4-OHT (#H6276, Sigma) at a final concentration of 20nM for 24h. Then flow cytometry analysis was performed as described above.

### Tissues and immunohistochemistry

Twenty-one cases of lung carcinoma (13 adenocarcinomas and 8 squamous cell carcinomas), 15 breast carcinoma, and 21 hepatocellular carcinoma (HCC) specimens were obtained from patients who had undergone initial surgery. The samples were fully encoded to protect patient confidentiality and were approved by the local research ethics committees at all participating sites.

Fresh tissues were embedded carefully at -20□ with OCT compound (optimal cutting temperature) in plastic mold, cut into 4-8 µm sections after equilibration in the cryostat chamber, and fixed with cold (20°) acetone for 15 min. For immunofluorescence double staining, the tissue sections were blocked and incubated with the primary antibodies at appropriate dilutions for 60 min, and subsequently incubated with anti-1gG-Cy3 (y-chain-specific) and anti-IgM-FIYC (µ-chain-specific) secondary antibodies, counterstained with DAPI, and mounted on glass slides with glycerol for microscopic analysis. For immunoperoxidase staining, the tissue sections were treated with 3% H₂O₂ for 30 min to block endogenous peroxidases, washed 3 times with PBS, and blocked with 2 % BSA. They were incubated with the primary antibody, then treated with peroxidase-labeled goat anti-mouse immunoglobulin antiserum (DAKO, Copenhagen, Denmark). Negative controls were performed with 2 % BSA in PBS instead of the mAbs. Anti-MUC mAb (PankoMab) was used in lung carcinoma specimens as positive controls in all batches. Color was developed with the peroxidase substrate diaminobenzidine. Counterstaining was performed with hematoxylin. Cell numbers were counted at 100x magnification with a Nikon microscope. The percentage of double positive cells was estimated on digital images.

### Sandwich ELISA

One million cells were treated with 1 ml of 1 % Triton-100 (in 100 mM sodium phosphate P_{H}, 7.5, 150 mM NaCl) containing a mixture of protease inhibitors (#539134, Calbiochem, Darmstadt. Germany) and homogenized with oscillation at 4 °C for 30 min. After centrifugation for 10 min at 15,000g in a centrifuge, the supernatants were taken. Ninety-six well polystyrene microplates were coated with a capture antibody against CD44 at 1 µg/ml working concentration at 4 ºC for 14 h. After blocking the remaining protein-binding sites with 5% BSA, 100 µl of antibody supernatants were added to the wells and incubated at room temperature for 2 h. Then the plates were incubated with CD176 mAb followed by peroxidase-labeled goat anti-mouse IgM antibody (µ-chain specific) (SouthernBiotech, - Birmingham, AL, USA). Color reaction was developed with o-phenylenediamine dihydrochloride (OPD) solution at room temperature. The reaction was stopped with 2.5 M sulfuric acid. Negative controls were performed with 2 % BSA in PBS instead of the mAbs. The optical density of each well was determined within 30 min using a microplate reader (Bio-Rad, Hercules, CA, USA) at 492nm.

### Statistical analysis

Data were analysed with either the Chi-square test or Fisher's exact probability test. Pearson's correlation analysis of enumeration data was also performed. A P<0.05 value was considered statistically significant.

### 2. Results

### Expression of CD44 and CD133 in lung, breast, and liver cancer cell lines

CD44 was detected on more than 60 % of the cells in most of the cancer cell lines. One cell line, HuH-7 (HCC), showed a low percentage of CD44-positive cells. CD133⁺ cells represented only a small subpopulation in the cell lines used in this study (less than 1 % of the cells) except for 801-D (giant cell lung carcinoma) and HuH-7 (HCC). These cell lines did consistently express CD133 at the surface of 15 % and 6 % of the cells, respectively.

### Expression of CD176 in lung, breast, and liver cancer cell lines

Through flow-cytometry and immunocytochemistry analysis with mAb NM-TF2, we found that CD176 was localized at the cellular surface and in the cytoplasm. The cell lines expressed CD176 at varying intensities: MDA231, MDA435 (breast adenocarcinoma), and HuH-7 (HCC) contained from 5 % to 30 % positive cells; SPC-A-1 (lung adenocarcinoma), 801-D (giant cell lung carcinoma), and HepG2 (HCC) revealed 30 % to 60 % positive cells; GLC-82 (lung adenocarcinoma), NCIH446 (small cell lung carcinoma), and MCF-7 (breast adenocarcinoma) had more than 60 % positive cells.

### Co-expression of CD176, CD44, CD133 in lung, breast, and liver cancer cell lines

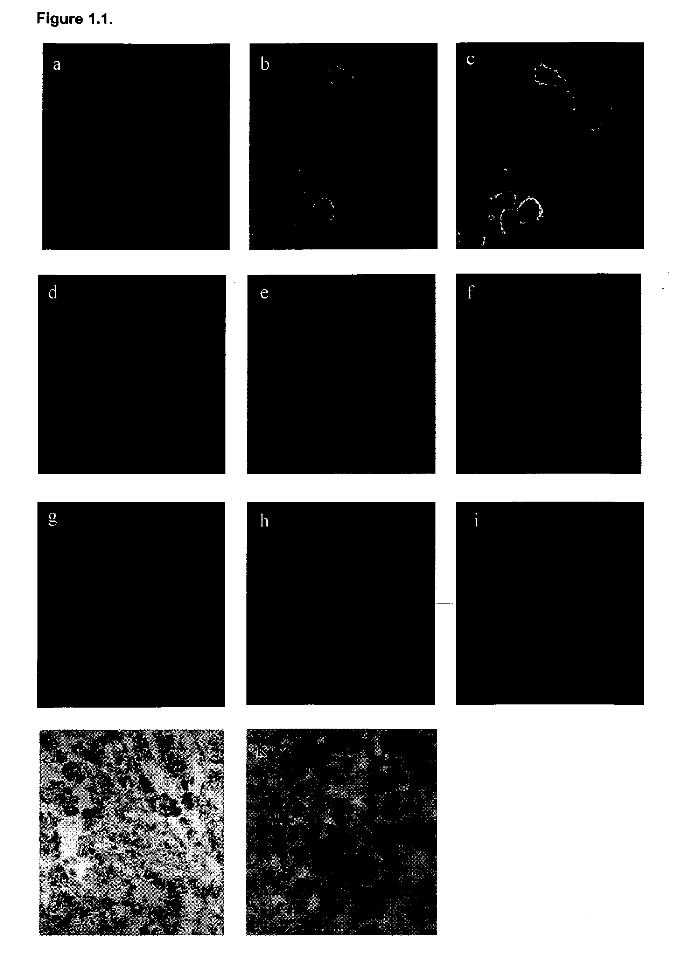

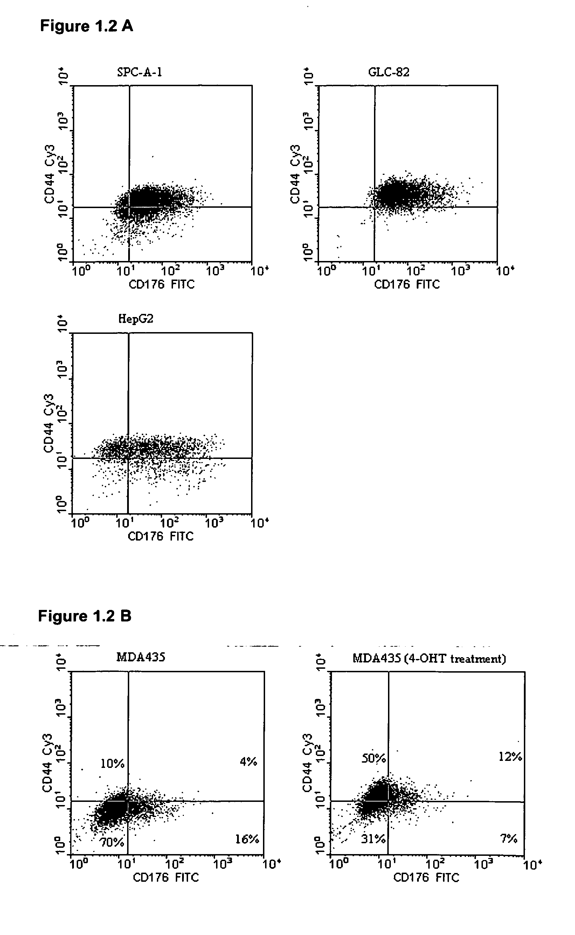

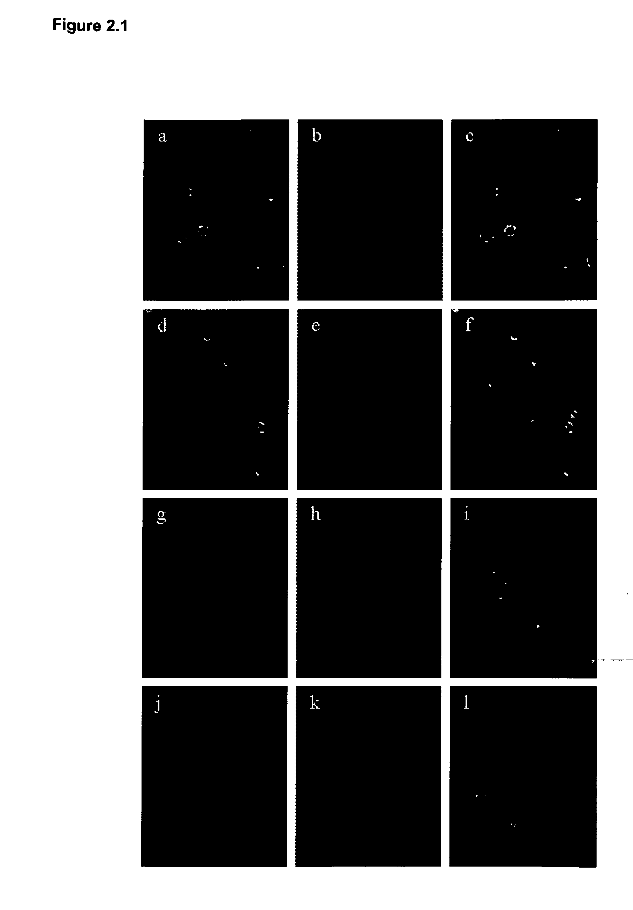

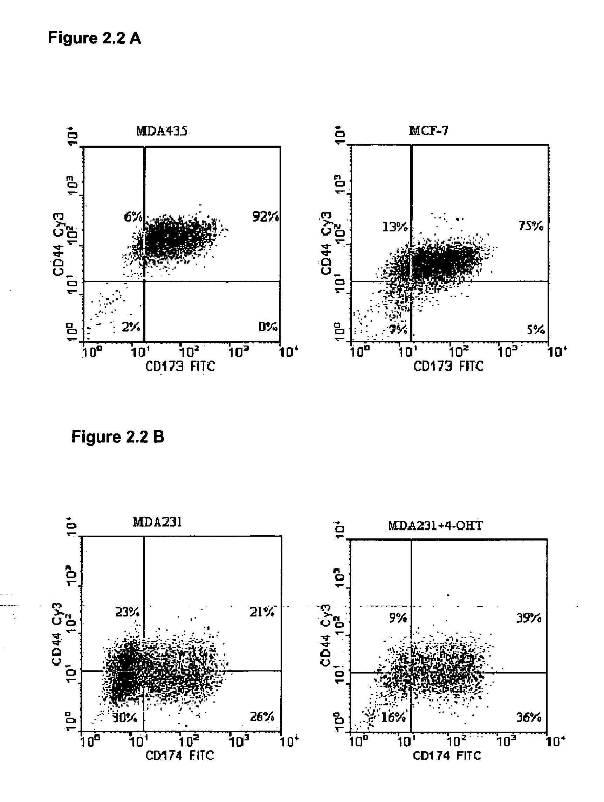

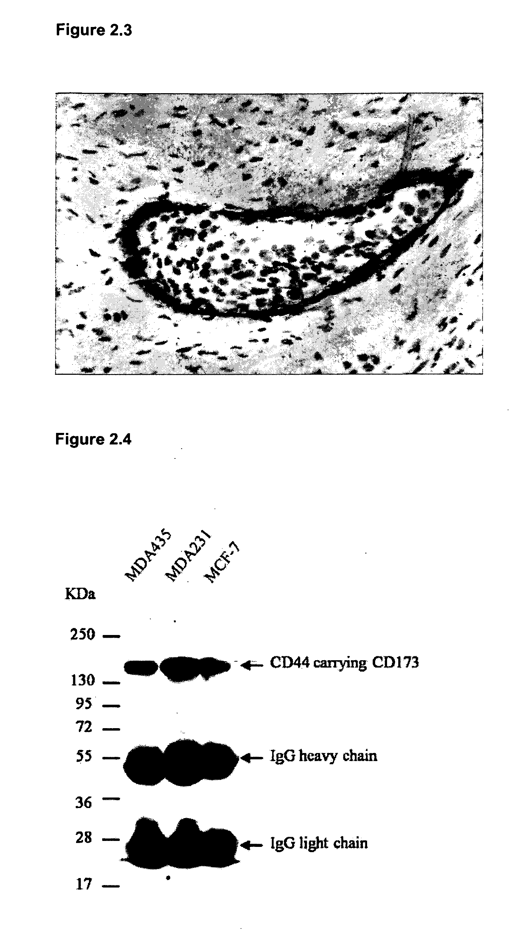

Since CD44 and CD133 are accepted markers of cancer-initiating cells (Chu et al. 2009: Bao et al. 2006: Ponnusamy and Batra 2008), we examined whether one or both are co-expressed with CD176. Double immunofluorescence staining experiments in cell lines demonstrated that CD44 and CD176 were located at the cellular surface and exhibited co-expression of single cells or cell clusters **(****Figure 1****, a-i).** Flow cytometry experiments revealed the following co-expression data. MCF-7 (breast adenocarcinoma) contained about 7 % of CD44⁺/CD176⁺ cells, SPC-A-1, 801-D and HepG2 contained from 30 % to 60 % of CD44⁺/CD176⁺ cells, and GLC-82 and NCIH446 had over 60 % of CD44⁺/CD176⁺ cells (see **Table 1.1,** **Figures 1****, 2).**

**Table 1.1: Flow-cytometric and immunofluorescent analysis of CD44 and CD176 expression in epithelial cancer cell lines**

| **Tissue derivation** | **Cell lines** | **CD44^{neg/} CD176^{neg}** | **CD44^{neg} CD176^{po8}** | **CD44^{p08} CD176^{neg}** | **CD44^{po8/} CD176^{po8}** |
|---|---|---|---|---|---|
| **Lung cancer** | | | | | |
| Adenocarcinoma | SPGA-1 | -6% | -20% | -5% | >60% |
| Adenocarcinoma | GLC-82 | <1% | -4% | -1% | >60% |
| Small cell carcinoma | NCIH446 | -10% | -8% | -17% | >60% |
| Giant cell lung carcinoma | 801-D | -23% | -6% | -46% | -23% |
| **Breast cancer** | | | | | |
| Adenocarcinoma | MDA-231 | >60% | -2% | -1% | -1% |
| Adenocarcinoma | MDA435 | >60% | 16% | -10% | -4% |
| Adenocarcinoma | MCF-7 | >60% | -4% | -24% | -7% |
| **Liver cancer** | | | | | |
| Hepatocellular carcinoma | HePG-2 | -6% | -17% | -23% | -54% |
| Hepatocellular carcinoma | HuH-7 | >60% | -13% | <1% | <1% |

| | | | | | |
|---|---|---|---|---|---|
| Scoring of percent of the positive cells | | | | | |

In contrast, most cell lines contained only few cells (less than 1 %) with the CD133*/CD176+ phenotype. As an exception, 801-D and HuH-7 had more than 5 % cells with the CD133^{+/}CD176⁺ phenotype (Table 1.2).

**Table 1.2: Flow-cytometric and immunoflurorescent analysis of CD133 and CD176 expression in cancer epithelial cells**

| **Tissue derivation** | **Cell lines** | **CD133^{neg/} CD176^{neg}** | **CD133^{neg/} CD176^{po8}** | **CD133^{po8/} CD176^{neg}** | **CD133^{Po8}/ CD176^{po8}** |
|---|---|---|---|---|---|
| **Lung cancer** | | | | | |
| Adenocarcinoma | SPC-A-1 | -24% | >60% | <1% | <1% |
| Adenocarcinoma | GLC-82 | -7% | >60% | <1% | <1% |
| Small cell carcinoma | NCIH446 | -15% | >60% | <1% | <1% |
| Giant cell lung carcinoma | 801-D | <1% | >60% | -11% | -4% |
| **Breast cancer** | | | | | |
| Adenocarcinoma | MDA-231 | >60% | -3% | <1% | <1% |
| Adenocarcinoma | MDA435 | >60% | -20% | <1% | <1% |
| Adenocarcinoma | MCF-7 | >60% | -11% | <1% | <1% |
| **Liver cancer** | | | | | |
| Hepatocellular carcinoma | HepG-2 | -29% | >60% | <1% | <1% |
| Hepatocellular carcinoma | HuH-7 | >60% | -13% | -2% | -4% |

| | | | | | |
|---|---|---|---|---|---|
| Scoring of percent of the positive cells | | | | | |

To assess whether CD44 and CD176 expression was affected simultaneously by exogeneous treatment, the estrogen receptor ligand tamoxifen (4-OHT) was added to breast cancer cells (24 h, 20 nM). Our results indicated that MDA231 and MCF-7 had no significantly increased number of CD44⁺/CD176⁺ co-expressing cells as shown by flow cytometry, whereas in MDA435 the percentage of CD44⁺/CD176⁺ cells was increased after treatment (Figure 2B).

### Expression of CD176 in lung carcinoma tissues

In this study the expression of CD176 in lung carcinoma tissues was investigated employing mAb NM-TF2. The tissues examined from 21 patients included 13 adenocarcinomas and 8 squamous cell carcinomas. CD176 was distributed at the cellular surface and in the cytoplasm. Examples are shown in **Figure 1****, j, k.** The percentage of CD176-positive cells in lung carcinomas is shown in Table 3. Four cases of lung carcinomas revealed over 50 % positive cells. These data confirm the expression of CD176 in lung cancer tissues in contrast to an earlier paper (Toma et al. 1999), a precondition for co-expression experiments. CD176 expression was compared with clinicopathological features by means of statistical analysis. Cases showing >5% of positive cells were defined as positive. The results show a correlation between the status of CD176 and tumor grade as well as metastasis in patients with lung carcinoma (P<0.05). However, there were no statistically significant correlations between the expression of CD176 and other clinicopathological features including patient age, sex, or histological subtypes (adenocarcinomas or squamous cell carcinomas).

### Co-expression of CD176 with CD44 or CD133 in lung, breast, and hepatocellular carcinoma tissues

The immunohistochemical data for CD44, CD133 and CD176 expression in the tissues in question are shown in Table 1.3. Similar to the cancer cell lines, CD44 was expressed strongly and diffusely in all carcinomas and at a high (more than 60 %) percentage. CD133* cells were found in most cases, but only in a subpopulation of cells (less than 10 % of the total cancer cells) within a given carcinoma tissue. Two cases of lung carcinomas, 3 of breast carcinomas and one HCC did not express CD133 at all. The CD133⁺ cells were present in a scattered pattern or in a clustered form, CD133 expression did not show any statistically significant correlation with sex, age or histological subtypes in lung carcinomas.

**Table 1.3: Evaluation of immunohistochemical positivity in lung breast, liver cancer tissues**

| **Cancer Types** | **% CD44"' cells** | | | | **% CD133"' cells** | | | | **% CD176^{po8} cells** | | | |
|---|---|---|---|---|---|---|---|---|---|---|---|---|
| | <5% | <30% | <60% | <100% | <5% | <30% | <60% | <100% | <5% | <30% | <60% | <100% |
| **Lung carcinoma** | 0/10 | 0/10 | 0/10 | 10/10 | 11/21 | 6121 | 1121 | 1121 | 5/21 | 9121 | 3/21 | 4/21 |
| **Breast carcinoma** | 0/15 | 1/15 | 6/15 | 8/15 | 10/15 | 2/15 | 0115 | 0/15 | 2115 | 12/15 | 1/15 | 0/15 |
| **HCC** | 0/21 | 0/21 | 9/21 | 12/21 | 11/15 | 3/15 | 0/15 | 0/15 | 10/21 | 7/21 | 4/21 | 0/21 |

| | | | | | | | | | | | | |
|---|---|---|---|---|---|---|---|---|---|---|---|---|
| No. of cases reactive/total no. of cases examined | | | | | | | | | | | | |

The co-expression of CD176 with CD44 or CD133 in the fresh clinical samples was examined. The double staining results in the cancerous tissues revealed that 5-30% of CD44* cells expressed CD176 simultaneously (see Table 1.4), and the most of CD133* cells also expressed CD176. However, we also noted that co-expression of CD976 with CD44 or CD133 was not seen in every cell positive for one of the markers. Some CD44⁺ and CD133⁺ cells did not express CD176 and *vice versa.* The HCC had significantly more cells with a CD133⁺CD176⁺ phenotype than either lung carcinoma or breast carcinoma. There was a statistically significant correlation between the status of CD176 and CD133 in lung carcinoma (P < 0.05). Correlations between clinicopathological features and the CD44^{*}CD176^{*} phenotype were not seen in this study.

**Table 1.4: Immunofluorescence analysis of CD44, C0133 and CD176 expression in lung, breast and liver cancer tissues**

| **Cancer Types** | **CD44neg /CD176neg** | **CD44neg** /**CD176pos** | **CD44pos** /**CD176neg** | **CD44pos** /**CD176pos** | **CD133neg** /**CD176neg** | **CD133neg** /**CD176po8** | **CD133pos /CD176neg** | **CD133pos /CD176pos** |
|---|---|---|---|---|---|---|---|---|
| **Lung carcinoma** | 1.5% | <1% | 30-60% | 5-30% | >60% | 5-30% | 1-5% | 1-5% |
| **Breast carcinoma** | 5-30% | 1-5% | 5-30% | 5-30% | >80% | 5-30% | 1-5% | 1-5% |
| **HCC** | 1-5% | 1-5% | 30-80% | 5-30% | >60% | 1-6% | 1-5% | 5-30% |

| | | | | | | | | |
|---|---|---|---|---|---|---|---|---|
| Scoring of percent of the positive cells | | | | | | | | |

### Detection of CD44 carrying CD176

Potential glycoprotein carrier molecules of CD176 were analyzed by a sandwich ELISA in several carcinoma cell lines. We used polystyrene microplates coated with CD44 as capture antibody to collect CD44 glycoprotein from whole-cell lysates. The plates were then incubated with NM-TF2 (CD176) as detection antibody. As shown in table 1.5, captured CD44 was found to react with the CD176 mAb in all (4) lung and (2) liver cancer cell lines as well as in 2 out of 3 breast cancer cell lines, indicating that CD44 apparently is a carrier of CD176 in these cases.

**Table 1.5: Reactive binding of CD176 in sandwich ELISA**

| **Tissue derivation** | **Cell lines** | **CD44+/CD176+** |
|---|---|---|
| **Lung cancer** | | |
| Adenocarcinoma | SPC-A-1 | + |
| Adenocarcinoma | GLC-82 | + |
| Small cell carcinoma | NCIH446 | (+) |
| Giant cell lung carcinoma | 801-D | (+) |

| **Breast cancer** | | |
|---|---|---|
| Adenocarcinoma | MDA-231 | - |
| Adenocarcinoma | MDA-435 | + |
| Adenocarcinoma | MCF-7 | (+) |

| **Liver cancer** | | |
|---|---|---|
| HepatoceHular carcinoma | HepG-2 | + |
| Hepatocellular carcinoma | HuH-7 | (+) |

| | | |
|---|---|---|
| Scoring according to OD490 minus blank as follows: ++; >0.5; + 0.2-0.5; (+), 0.1-0.2; — <0.1. | | |

**Table .6: CD44⁺/CD176⁺ cells increased in breast cancer cells following 4-OHT treatment**

| **Tissue derivation** | **Cell lines** | **CD44⁻/CD176⁻** | **CD44⁻/CD176⁺** | **CD44^{-/}CD176⁺** | **CD44⁺/C178⁺** |
|---|---|---|---|---|---|
| **Breast cancer (Control)** | | | | | |
| Adenocarcinoma | MDA-231 | >60% | -2% | -1% | -1% |
| Adenocarcinoma | MDA435 | >60% | -16% | -10% | -4% |
| Adenocarcinoma | MCF-7 | >60% | -4% | -24% | -7% |

| **Breast cancer (4-OHT treatment)** | | | | | |
|---|---|---|---|---|---|
| Adenocarcinoma | MDA231 | >60% | -13% | -1% | -2% |
| Adenocarcinoma | MDA435 | -31% | -7% | -50% | -12% |
| Adenocarcinoma | MCF-7 | >60% | -8% | -20% | -8% |

| | | | | | |
|---|---|---|---|---|---|
| Scoring of percent of the positive cells | | | | | |

### 3. Discussion

Numerous publications have led to a general acceptance of the view that initiation, maintenance, and dispersion of most if not all tumor types is essentially due to a small population of cells called cancer-initiating cells or cancer stem cells (Chu et al. 2009: Bao et al. 2006).

Cancer stem cells exhibit the propensity to differentiate into actively proliferating tumor cells. They clearly differ from the majority of cells of the tumor mass, but they should be (co)targeted in cancer therapies. A great number of more or less specific markers of cancer-initiating cells have been described during recent years. The crux of these markers is that there are too many of them, and that they are not sufficiently consistent. Among the markers most widely accepted is CD44 (Ponnusamy and Batra 2008; Shipitsin et al. 2007). Another membrane glycoprotein, CD133, has also been proposed to be capable of identifying a cancer initiating population in brain, colon, lung, and other solid tumors (Tirino et al. 2008). CD133 is an independent prognostic marker that correlates with poor overall survival in patients with malignancies (host et al. 2008). However, a recent report showed that CD133 negative cells were also capable of long-term tumorigenesis in NOD/SCID mice (Shmelkov et al. 2008).

In this study underlying the present invention the expression of CD44 and CD133 was evaluated in cell lines and in clinical samples from lung, breast, and liver cancer. It was found that the large majority of tumor cells stained positively for CD44. In contrast, most of the cancer cell lines studied revealed low CD133 expression. CD133 was consistently expressed in lung, breast and liver cancer tissues at varying intensities following staining. Other authors have reported that CD133 was expressed on about 2.5 % and 6-29 % of the total number of tumor cells in colon cancer (Ricci-Vitiani et al. 2007), and non-small cell lung cancer (Tirino et al. 2009) and brain tumors (Singh et al. 2004), respectively.

Then the co-expression of these two markers of cancer-initiating cells, CD44 and CD133, with CD176 was analysed. A major percentage of cells was found as co-expressing CD176 and CD44, especially in lung carcinoma cell lines and in HepG-2, and a significant number in all other examined cell lines. In the examined lung, breast and liver cancer tissues the number of cells co-expressing CD176 and CD44 amounted to 5-30 %. The number of cells co-expressing CD176 and CD133 was much lower in all cases (as was the total number of CD133 expressing cells), but co-expressing cells were never completely absent.

The data presented here demonstrates that CD176 is not only expressed on mature cancer cells, but also on cancer-initiating cells of solid tumors. This adds additional weight on CD176 as an attractive therapeutic target. It was also analysed whether the number of CD176⁺ breast cancer cells is enhanced after treatment with tamoxifen (4-OHT). Tamoxifen was reported to induce G0/G1 growth arrest and to inhibit the proliferation of breast cancer cells, A recent study showed that tamoxifen treatment increased the number of mammary cancer stem cell-like cells (Mani et al. 2008). In our study, tamoxifen treatment of breast cancer cells enhanced the CD44⁺/CD176⁺ phenotype in one out of 3 cell lines (MDA-435). According to the cancer stem cell theory, recurrences and metastases of cancer depend on cancer-initiating cells. The existence of a population of such cells with properties different from the tumor mass may explain why conventional therapies, e.g. treatment with tamoxifen, are only able to suppress cancer but often cannot completely eradicate it. On the contrary, this treatment may even enhance the number of cancer-initiating cells (Mani et al. 2008). Theoretically, the elimination of cancer-initiating cells could prevent recrudescences of tumors. The development of new therapeutic approaches to target cancer-initiating cells may therefore have a profound impact on cancer therapy. Thus, the identification of CD176 on cancer-initiating cells of solid tumors is important for a future application of CD176-based immunotherapies.

Demasking of CD176 seems to be a selective process which involves only a few among all possible candidate glycoproteins present at the cell membrane. The most prominent carrier molecule of CD176 identified in epithelial cells so far is the polymorphic epithelial mucin MUC-1, for example in colorectal carcinoma (Barr et al. 1989; Cao et al. 1997; Baldus et al. 1998). Here, it was determined whether CD44 is a carrier molecule for core-1 in the analysed cells. To this end we applied a special sandwich ELISA and examined with it lung, breast and liver cancer. Our data suggest that CD176 was remarkably carried by CD44 in tumors other than colorectal carcinomas. The CD176 antigen has been found to be a useful marker of prognosis in lung patients (Takanami 1996), but there remain conflicting reports on its expression in this tissue (Toma et al. 1999). In our study, we re-examined this issue by employing the monoclonal antibody NM-TF2, which is well characterized and suited for immunohistochemistry and other techniques. High CD176 expression was observed in most lung cancer cell lines. In clinical lung cancer samples we also found that more than 50 % of the cases were CD176⁺ above the cut off value (5% positive cells).

In summary, CD176 (Thomsen-Friedenreich antigen, core-1) expression was observed in human lung, breast and liver carcinomas and in cell lines derived from these malignancies. Co-expression of CD44 and CD176, as well as of CD133 and CD176 in human lung, breast and liver carcinoma demonstrated that CD176 was not only expressed on mature cancer cells, but also on cancer-initiating cells. This makes CD176, which is almost absent in normal and benign adult human tissues (Cao et al. 1996), an even more promising target for tumor therapies.

### II. Co-expression of CD173 (H2) and CD174 (Lewis Y) with CD44 on breast cancer-initiating cells

### 1. Material and methods

### Cell lines and cell culture

The breast adenocarcinoma cell lines MDA-MB-231, MDA-MB-435, and MCF-7 were used in this study. These cell lines were routinely cultured in Dulbecco's Modified Eagle Medium (DMEM) containing 10% fetal calf serum. The cells were grown at 37 °C in an atmosphere of 5% CO₂/95% air and close to 100% relative humidity.

### lmmunocytochemistry

The cells were plated onto polylysine (Sigma, Saint Louis, MO, USA)-coated slides in culture medium overnight. Thereafter the medium was carefully aspirated, and the slides were air-dried. Wrapped slides could be stored at -80U until use. For immunocytochemistry, cells were fixed with cold (-20°C) acetone for 15 min, blocked with 2% bovine serum albumin (BSA) for 30 min, incubated with CD44 mAb (G44-26, mouse IgG2b, BD Biosciences, Franklin Lakes, NJ, USA) together with mAbs CD173 (A46-BIB10, mouse IgM, Karsten 1988) or CD174 (A70-C/C8, mouse IgM), both from Glycotope GmbH (Berlin, Germany) for 60 min. In addition, the CD133 antibody (ANC9C5, Ancell, Bayport, MN, USA) was used. The slides were subsequently incubated with a mixture of fluorescein isothiocyanate (FITC)-conjugated anti-mouse IgM (µ chain-specific) (F9259, Sigma) and Cy3-conjugated goat anti-mouse IgG (y chain-specific) (#69732, Jackson Laboratories, West Grove, PA, USA). Counterstaining was performed with 4',6-diamidino-2-phenylindoie dihydrochloride (DAPI) (Beyotime Biotechnology, Jiangsu, China). Negative controls were incubated with mouse serum instead of the specific mAb. The slides were mounted with glycerol and analysed by fluorescence microscopy.

### Flow cytometry analysis

Cell suspensions from established cell lines were prepared at a density of 1x10⁶ cells/100µl. They were washed twice with phosphate buffered saline (PBS) containing 2% BSA and incubated with primary antibodies at 4° for 20 min, followed by anti IgG-Cy3 (y chain-specific) and anti IgM-FITC (µ chain-specific) in appropriate concentrations at 4° for 20 min. Flow cytometry was performed on a FACScan (BD Biosciences, Franklin Lakes, NJ, USA). Collected data from 10,000 cells and WinMDl software were used for the analysis of FACS datafiles.

For 4-hydroxytamoxifen treatment, MDA-MB-231, MDA-MB-435 and MCF-7 breast adenocarcinoma cell lines were exposed to 4-OHT (#H6278, Sigma) at a final concentration of 20 nM for 24 h. Then flow cytometry analysis was performed as described above.

### Immunoprecipitation

One million cells were treated with 1ml of 1% Triton-100 in 50 mM Tris-HCl pH 8.0, 150 mM NaCI containing a mixture of protease inhibitors (#539134, Calbiochem, Darmstadt, Germany), and homogenized with oscillation at 4° for 30 min. After centrifugation for 10 min at 15,000 g, supernatants were taken. Protein G-agarose beads (#P-4691, Sigma) were pre-cleared to remove non-specific binding material, and then incubated for 4h with anti-CD44 antibody at 4° on a shaker. The protein G-agarose beads were added to the supernatants and incubated on an end-over-end mixer overnight at 4°. The beads were collected by centrifugation and washed. The pellets were resuspended in SDS-polyeacrylamide gel electrophoresis (PAGE) sample buffer and boiled for 5 min. The immunoprecipitates were subsequently separated on polyacrylamide gels (8 %) and probed using anti-CD173 and anti-CD174 antibodies in Western blotting.

### Sandwich ELISA

Ninety-six well polystyrene microplates were coated with a capture antibody against CD44 at 1 µg/ml in PBS at 4° for 14 h. After blocking with 5 % BSA. 100 µl of the supernatants prepared as described above were added to the wells and incubated at room temperature for 2 h. Then the plates were incubated with CD173 or CD174 mAb followed by peroxidase-laboled goat anti-mouse 1gM antibody (µ-chain specific) (Southern Biotech, Birmingham, AL, USA). Color reaction was developed with o-phenylenediamine dihydrochloride (OPD) solution at room temperature. The reaction was stopped with 2.5 M sulfuric acid. Negative controls were performed with 2 % BSA instead of the mAbs. The optical density of each well was determined within 30 min using a microplate reader (Bio-Rad, Hercules, CA, USA) at 492 nm.

### Tissues and immunohistochemistry

Fifteen breast carcinoma specimens were obtained from patients who had undergone initial surgery. The samples were fully encoded to protect patient confidentiality, and the study and study protocols were approved by the local research ethics committees at all participating sites.

The fresh tissues were embedded carefully at -20 °C with OCT compound (optimal cutting temperature) in plastic mold, cut into 4-8 µm sections after equilibration in the cryostat chamber, and fixed with cold (20 °C) acetone for 15 min. Then immunofluorescence double stainings were performed as described above. Cell numbers were counted at 100x magnification with a Nikon microscope. The percentage of double-positive cells was estimated in digital pictures.

In 3 additional cases of breast intraductal carcinoma, we also performed immunoperoxidase staining. The tissue sections were treated with 3 % H₂O₂ for 30 min to suppress endogenous peroxidases, washed 3 times with PBS, and blocked with 2 % BSA. They were incubated with CD173 antibody, then treated with peroxdase-labeled goat ant-mouse immunoglobulin antiserum (DAKO, Copenhagen. Denmark). Negative controls were performed with 2 % BSA instead of the CD173 antibody. Color was developed with diaminobenzidine. Counterstaining was performed with hematoxylin.

### Statistical analysis

The statistical analyses were performed using unpaired t test, Fisher's exact test, or the Spearman correlation test. Data were expressed as means ± standard error of the mean (SEM). P < *0.05* was considered statistically significant.

### 2. Results

### Expression of CD173 and CD174 on breast cancer cell lines

Flow cytometric analysis and immunohistological staining revealed that CD173 and CD174 were localized at the cellular surface (Figure 1a). The breast cell lines showed frequent CD173 and CD174 expression: MDA-MB-231, MDA-MB-435 and MCF-7 contained about 27 %, 92 % and 80 % of CD173 positive cells, as well as about 44 %, 57 % and 72 % of CD174 positive cells, respectively.

### Co-expression of CD44 with CD173 or CD174 in breast cancer cell lines

lmmunocytological staining experiments were performed to investigate the co-expression of CD173 or CDC 74 with CD44 on breast cancer cells. We observed in many cells an overlay of CD44 staining with CD173 and CD174; an example is given in Figure 1a-c. Semiquantitative data from flow cytometry experiments are shown in **Figure 2A,** and summarized in **table 2.1.**

**Table 2.1: Flow-cytometric analysis of CD44, CD173 and CD174 expression in breast carcinoma cell lines**

| **Cell lines** | **CD44pos** | **CD173pos** | **CD174pos** | **CD44pos/CD173pos** | **CD44pos/CD174pos** |
|---|---|---|---|---|---|
| **MDA-MB-435** | >60% | >60% | -57% | >60% | -57% |
| **MDA-MB-231** | -44% | -27% | -44% | -17% | -21 % |
| **MCF-7** | >60% | >60% | >60% | >60% | -46% |

To assess whether CD44, CD173 and CD174 expression are simultaneously affected after exogeneous treatment, we treated the breast cancer culture cells with 4-OHT. In semiquantitative flow cytometric analysis, 4-OHT treatment increased the percentage of CD44⁺CD173⁺ and CD44⁺CD174⁺ cells in MDA231 (P < 0.05) **(Figure 2B),** but not in MDA-MB-435 and MCF-7 cells (P > 0.05, data not shown).

### Co-expression of CD44 or CD133 with CD173 or CD174 in breast carcinoma tissues

Among the examined fifteen breast carcinoma tissues, CD44, CD173 and CD174 staining was also predominantly observed at the cell membrane. CD44, CD173 and CD174 positive cells were present in a scattered or clustered pattern, which varied among individuals. CD173 and CDC174 positive cells were found in most cases. The mean percentage of CD173 and CD174 positive cells was 51 % and 52 % of the total cancer cells, respectively. CD44 showed strong immunoreactivity (positively stained carcinoma cells amounted to 53 %) in all cases. Interestingly, cases with increased CD173 and CD174 expression correlated with raised CD44 expression (P<0.05). Most importantly, more than 95 % of CD173 and CD174 positive cells, respectively, co-expressed the CD44 antigen **(****Figure 1****, d-i),**

CD133 is also a marker of cancer-initiating cells. Therefore, we asked whether CD173 or CD174 was co-expressed with this marker in breast carcinoma tissues. We found that CD133 was indeed co-expressed with CD173 and CD174, although at a lower percentage (Figure 1j-l). In addition, we found that cases with increased CD173 and CD174 expression correlated with raised CD133 expression *(P<0.05)* (data not shown).

### Expression of CD173 on basal cells of intraductal breast carcinomas

In 3 cases of intraductal breast carcinomas, CD173 mAb stained the basal cells of the remaining duct walls (Figure 3). In normal ducts of transitional tissues of the same sections. the surrounding ductal (mainly basal) cell layer was only occasionally positive for CD173. The expression of CD173 in these cells was independent of the ABH blood group type and of the secretor status of the individual (data not shown).

### Evidence for CD44 carrying CD173 or CD174

Potential glycoproteins carrying CD173 or CD174 were analyzed in 3 breast carcinoma cell lines by immunoprecipitation and in a sandwich ELISA. The CD44 immunoprecipitate from the lysates of the three cell lines was subjected to immunoblot analysis using mAbs CD173 and CD174. Both antibodies stained the CD44 band (Figure 4).

A sandwich ELISA with anti-CD44 as capture antibody followed by mAbs CD173 and CD174, both antibodies scored positive in all 3 cell lines examined, indicating that CD173 and CD174 epitopes are expressed on the CD44 molecule (table 2.2).

**Table 2.2: Binding of CD173 and CD174 antibodies in an sandwich ELISA with CD44 as catcher antibody in 3 breast cancer cell lines**

| **Tissue derivation** | **Cell lines** | **CD44pos/CD173pos** | **CD44pos/CD174pos** |
|---|---|---|---|
| **Adenocarcinoma** | MDA-MB-435 | + | (+) |
| **Adenocarcinoma** | MDA-MB-231 | + | + |
| **Adenocarcinoma** | MCF-7 | + | (+) |

| | | | |
|---|---|---|---|
| Score according to OD₄₉₀ values minus blank as follows: ++, >0.5; +, 0.1-0.5; (+), 0.05-0.1; -, <0.05 | | | |

### 3. Discussion

The existence of cancer-initiating cells is well documented in brain, lung and breast cancers (Tirino, 2008). They are functionally defined as self-renewing, quiescent and multi-potent cells that are able to multi-lineage differentiation. According to the cancer stem cell theory, recurrences and metastases of cancer depend on cancer-initiating cells (Gilbert, 2009). Since cancer-initiating cells or cancer stem cells clearly differ from the majority of cells of the tumor mass, studying the expression and function of surface molecules on cancer-initiating cells is an important aspect of tumor biology.

In this study, we examined whether CD173 and/or CD174 are co-expressed with the cancer-initiating cell marker CD44 in breast carcinomas.

The present study indicates that CD44 together with CD173 or CD174 are located at the cell surface and exhibit co-expression in a significant proportion of cultured breast cancer cells and in tissue specimens taken from breast cancer. Tamoxifen was reported to increase the number of mammary cancer stem cells (Mani, 2008). In this study, the number of CD44⁺/CD173⁺ or CD44^{*}/CD¹⁷⁴+ breast cancer cells could be enhanced in cultured cells after tamoxifen (4-OHT) treatment. Therefore, we conclude that CD44 is co-expressed with CD173 and CD174 in breast cancer.

An interesting observation was the strong staining for CD173 of remaining duct wall cells in cases of intraductal breast carcinomas. This indicates proliferative activity of stem cell-like basal cells under the mechanical stress of the expanding tumor mass. Since H and LeY antigens are developmentally regulated antigens, this phenomenon might also be indicative of an ongoing epithelial-mesenchymal transition (EMT) of these cells.

Type 2-based ABH oligosaccharides are carried on several different glycoproteins and glycolipids (Hakomori, 1981). In epithelial ovarian cancer, the major carrier proteins of CD174 are CA125 and MUC1 (Yin, 1996). In CD34⁺ hematopoietic stem cells, the major carrier of CD173 and CD174 is a 170 kDa glycoprotein (Cao. 2001). CD44 is also a carrier of H antigens (Rapoport. 1999). Here we have used a sandwich ELISA and immunoprecipitation to demonstrate that CD44 is a major carrier of CD173 and CD174 in breast cancer cells.

It is believed that CD173 and CD174 structures on glycoprotein expressed by carcinomas contribute to adhesion, cell aggregation, invasion, and metastasis. CD174 Is Involved in early cell-cell contacts during tumor associated angiogenesis (Moshler, 2008). Histochemistry alone cannot decide what the function and significance of the expression of CD173 and CD174 on cancer-initiating cells is. However, a pathophysiological significance of the expression of CD173 and CD174 in carcinomas has been demonstrated. CD173 and CD174 are apparent markers of the degree of malignancy in cancer patients (Fujitani, 2001; Steplawska-Mazur, 2000). Higher expression of CD173 and CD174 was more often found In patients with high grade and poor prognosis compared to those with better prognosis (Baldus, 2006). In lymph node negative breast carcinomas, over-expression of CD174 was associated with significantly decreased patient survival (Madjd, 2005). Increased tumorigenicity mediated by α1-2 fucosylation is associated with increased resistance to apoptosis and escape from Immune control (Goupille, 2000).

Failure of current cancer therapies may be ascribed to the inefficacy of drugs on potentially quiescent cancer stem cells. Treatment strategies therefore need to consider the presence of cancer stem cells, The high expression of CD173 and especially of CD174 on the surface of cancer stem cells in breast carcinomas suggests that these antigens are promising targets for antibody-mediated diagnosis and therapy. More recent studies have demonstrated that the administration of low doses of anti-CD174 mAb may lead to an effective anti-tumor response, even without induction of TNF-α release (Dettke, 2000). and anti-CD174 antibody conjugated with doxorubicin is presently under evaluation in the therapy of epithelial tumors (Tolcher, 1999).

In summary, the identification of CD173 and CD174 on cancer stem cells offer new opportunities in therapy that target cancer-initiating cells in particular in the prevention of relapse.

## Claims

1. A binding agent specifically binding a tumor-associated carbohydrate antigen for use in the treatment of cancer stem cells expressing said tumor-associated carbohydrate antigen.

2. The binding agent according to claim 1, wherein the tumor-associated carbohydrate antigen is selected from the group consisting of CD176, CD175, CD175s, CD174, CD173, and CA19-9.

3. The binding agent according to claim 1 or 2, wherein the cancer stem cells have one or more of the following characteristics:
a) the cancer stem cells express at least one stem cell marker which is a glycoprotein that carries said tumor-associated carbohydrate antigen;
b) the cancer stem cells express one or more stem cell markers selected from the group consisting of CD34, CD44, CD44v6, CD133 and CD164.
c) said tumor-associated carbohydrate antigen is carried on a tumor-associated glycoprotein expressed by the cancer stem cells;
d) said tumor-associated carbohydrate antigen is tumor-specific; and/or
e) said tumor-associated carbohydrate antigen is expressed predominantly or exclusively on cancer stem cells but not on cancer cells that are no cancer stem cells.

4. The binding agent according to one of the claims 1 to 3, wherein the cancer stem cells are of a cancer having one or more of the following characteristics:
a) It Is a solid tumor;
b) It is a leukemia;
c) It is a multiple myeloma or lymphoma;
d) It is a tumor of epithelial origin; and/or
e) it is a tumor selected from the group consisting of lung, breast, liver, ovarian, gastrointestinal, pancreatic, prostate, cervical and head and neck cancer.

5. The binding agent according to claim one of the claims 1 to 4, wherein said binding agent has one or more of the following characteristics,
a) it is an antibody or an antigen-binding fragment or derivative of an antibody;
b) it is a human, murine, humanized, or chimeric antibody or a respective antigen-binding fragment or derivative;
c) it is a single-chain antibody fragment, a multibody, a Fab fragment, and/or an immunoglobulin of the IgG, IgM, IgA, IgE, IgD isotypes and/or subclasses thereof;
d) it is an antibody or an antigen-binding fragment or derivative of an antibody having one or more of the following characteristics:
i. it mediates ADCC and/or CDC of cancer -cells;
ii. it induces and/or promotes Apoptose of cancer cells;
iii. it inhibits proliferation of the target cells of cancer cells;
iv. it induces and/or promotes phagocytosis of cancer cells and/or
v. induces and/or promotes the release of cytotoxic agents;
e) it specifically binds said tumor-associated carbohydrate antigen, which is a tumor-specific carbohydrate antigen; and/or
f) it does not bind an antigen, in particular a carbohydrate antigen, expressed on non-cancer cells, non-tumor cells, benign cancer cells and/or benign tumor cells.

6. The binding agent according to claim 5, wherein said antibody or said functionally active fragment or derivative of said antibody is selected from the group consisting of:
a) a CD176 specific antibody or an antigen-binding fragment or derivative thereof, which preferably has at least one of the following characteristics:
i) it comprises at least one complementarity determining region (CDR), preferably at least two CDRs, more preferably all three CDRs. selected from the group consisting of CDRH1 having the amino acid sequence of SEQ ID No. 1, CDRH2 having the amino acid sequence of SEQ ID No. 2 or 3, and CDRH3 having the amino acid sequence of SEQ ID No. 4 or 5 or 6;
ii) it comprises at least one complementarity determining region (CDR), preferably at least two CDRs, more preferably all three Corps, selected from the group consisting of CDRL1 having the amino acid sequence of SEQ ID No. 7 or 8 or 9, CDRL2 having the amino acid sequence of SEQ ID No. 10 or 11, and CDRL3 having the amino acid sequence of SEQ ID No. 12 or 13;
iii) it comprises a heavy chain variable region containing the amino acid sequence of any one of SEQ 10 Nos. 46 to 79;
iv) it comprises a light chain variable region containing the amino acid sequence of any one of SEQ ID Nos. 80 to 94;
v) it does not specifically interact with Galal-3GaINAca, Galαl-3GalNAcβ, GaINAcα, Neu5Aca2-3Ga1β1-3GaINAcα, Galβ1-3(Neu5Acα2-6)GaINAca, GlcNAcβ1-2Gαβ1-3GaINAcα, GlcNAcαl-3Galβ1-3GalNAcα, GaINAcα1-3Galβ and/or 3'-O-Su-Galβ1-3GalNAcα under physiological conditions;
b) an antibody or an antigen-binding fragment or derivative thereof which shows cross-specificity with an antibody as defined in (a):
c) a CD173 specific antibody or an antigen-binding fragment or derivative thereof;
d) a God174 specific antibody or an antigen-binding fragment or derivative thereof;
e) a CD175 specific antibody or an antigen-binding fragment or derivative thereof;
f) a CD 175s specific antibody or an antigen-binding fragment or derivative thereof and/or
g) a GA19-9 specific antibody or an antigen-binding fragment or derivative thereof.

7. A pharmaceutical composition comprising a binding agent specifically binding a tumor-associated carbohydrate antigen for use in the treatment of cancer stem cells expressing said tumor-associated carbohydrate antigen, wherein the binding agent, the cancer stem cells and the tumor-associated carbohydrate antigen have one or more characteristics as are defined in any of claims 1 to 6.

8. The binding agent according to any one of claims 1 to 6 or the pharmaceutical composition according to claim 7, for use in therapy for the reduction of tumor size, elimination of malignant cells, the prevention of metastasis, the prevention of relapse; the reduction or killing of disseminated cancer, the prolongation of survival and/or the prolongation of the time to tumor respectively cancer progression.

9. A method for identifying a cancer comprising cancer stem cells that is susceptible to treatment with a binding agent that specifically binds a tumor-associated carbohydrate antigen wherein said treatment effects the cancer stem cells, comprising determining whether a cancer sample obtained from a patient comprises cancer stem cells that express the tumor-associated carbohydrate antigen the binding agent is specific for, wherein the presence of said tumor-associated carbohydrate antigen on cancer stem cells indicates that the cancer is susceptible to treatment with the binding agent that specifically binds said tumor-associated carbohydrate antigen and wherein said treatment effects the cancer stem cells.

10. A method for diagnosing, staging and/or prognosing cancer and/or monitoring the susceptibility to treatment, comprising the step of analyzing the expression of a tumor-associated carbohydrate antigen on cells in a sample isolated from a patient, wherein the presence of cells expressing the tumor-associated carbohydrate antigen indicates the presence of cancer stem cells in said sample.

11. The method according to claim 9 or 10; wherein the sample from said patient is stained with a binding agent specifically binding the tumor-assodated carbohydrate antigen.

12. The method according to any one of the claims 9 to 11, wherein the tumor-associated carbohydrate antigen is selected from the group consisting of CD176, CD175, CD175s, CD1 74, CD173, and CA19-9.

13. The method according to any one of the claims 9 to 12, wherein the presence of cells co-expressing at least one second cancer stem cell marker indicates the presence of cancer stem cells.

14. The method according to any one of the claims 9 to 13, wherein the co-expression of at least one additional glycoprotein cancer stem cell markers is tested, that is selected from the group consisting of CD34, CD44, CD44v6, CD133, and CD164.

15. The method according to any one of claims 9 to 14, wherein the analysis of the staining pattern provides the relative distribution of cancer stem cells, which distribution predicts the tumorgenicity of the cancer.

16. A composition of mammalian cancer stem cells, wherein at least 50%, preferably at least 75% of the cells in the composition are cancer stem cells which express a tumor-associated carbohydrate antigen.

17. A kit for use in a method according to any one of the claims 9 to 15 or 20, comprising a binding agent which specifically binds a tumor-associated carbohydrate antigen and instructions for use in a method according to one or more of the claims 9 to 15 or 20.

18. A method of screening a candidate therapeutic agent for effectiveness against a cancer stem cell expressing a tumor-associated carbohydrate antigen, the method comprising:
a) Contacting said agent with the cell composition of claim 16, and
b) Determining the effectiveness of said agent against said tumor-associated carbohydrate antigen positive cancer cells.
